# EUROPEAN PATENT SPECIFICATION

(11) **EP 1 064 384 B1**
(45) Date of publication and mention of the grant of the patent: **29.09.2010**
(21) Application number: 99911926.6
(22) Date of filing: 22.03.1999
(51) Int. Cl.: C12N 15/29, C12N 15/82, C12N 15/11, C12N 5/10, C07K 14/415, A01H 5/00, A01H 5/10

(54) **SIGNAL TRANSDUCTION PROTEIN INVOLVED IN PLANT DEHISCENCE**
SIGNALÜBERTRAGUNGSPROTEIN MIT FUNKTION IN PFLANZLICHER DEHISZENZ
PROTEINE TRANSDUCTRICE DE SIGNAUX, IMPLIQUEE DANS LA DEHISCENCE DE PLANTES

(30) Priority: 20.03.1998 GB 9806113
(43) Date of publication of application: 03.01.2001
(73) Proprietor: BIOGEMMA UK LIMITED, Cambridge CB4 0GZ (GB)
(72) Inventor: WYATT, Paul, Cambridge CB4 0GZ (GB); ROBERTS, Jeremy, Alan, Univ. of Nottingham, Loughbourough, Leicestershire LE12 5RD (GB); WHITELAW, Catherine, Loughbourough, Leicestershire LE12 5RD (GB)
(74) Representative: Flesselles, Bruno F.G.
(86) International application number: PCT/GB1999/000905
(87) International publication number: WO 1999/049046

(56) References cited:
- WO-A-94/23043
- WO-A-96/30529
- WO-A-97/13865
- NELSON D E ET AL: "Abundant accumulation of the calcium-binding molecular chaperone calreticulin in specific floral tissues of Arabidopsis thaliana." PLANT PHYSIOLOGY, (1997 MAY) 114 (1) 29-37. , XP002111979
- WHITELAW C A ET AL: "An mRNA encoding a response regulator protein from Brassica napus is up-regulated during pod development" JOURNAL OF EXPERIMENTAL BOTANY, (MAR 1999) VOL. 50, NO. 332, PP. 335-341., XP002111980

## Description

This invention relates to novel plant nucleic acid sequences and proteins. The sequences and proteins are useful in the control of plant dehiscence and in the production of male sterile plants.

The production of seed is an important developmental process in all higher plants. In oilseed rape (*Brassica napus*), following abscission of floral parts, pods or siliques are formed which contain 15-30 seeds. Around 50-70 days after anthesis (DAA) the pods become susceptible to shatter, a process that serves to expel the mature seeds into the surrounding environment. In the days leading to dehiscence, an array of anatomical, molecular and biochemical changes take place, thus preparing both seed and pod for the event. Shatter eventually occurs as a result of a combination of factors including: the creation of tensions within the pod between the lignified valve edge cells of the endocarp and the unlignified dehiscence zone (DZ) cells, weakening of the DZ cell walls by hydrolytic enzyme activity and ultimately due to physical forces such as strong winds or harvesting machinery.

Pod development in *B. napus* can be segmented into three stages. In the first stage, which occurs 0-20 DAA, the newly formed siliques, consisting of two seed-containing carpels separated by a false septum and a replar region, grow to their full length of around 10cm. The seeds begin to grow when the pods are virtually full size [Hocking and Mason, 1993]. Between 10 and 20 DAA the cells in the replar region begin to differentiate into replar cells, large valve edge cells and form a distinct region, 1-3 cells wide, comprising the DZ [Meakin and Roberts, 1990a].

The second stage occurs between 20 and 50 DAA. From 20 DAA, in conjunction with termination of pod elongation, secondary cell wall material is deposited in the valve edge cells, and the replar cells become increasingly lignified. The DZ cells do not exhibit thickening of the cell wall. A progressive shrinkage and loss of organelles is apparent in the DZ cells from 40 DAA onwards and eventually these cells separate completely due to hydrolysis of the middle lamella [Meakin and Roberts, 1990a]. In the final stage of pod development, which occurs 50-70 DAA, the lignified cells undergo senescence and the necessary tensions are created so that the desiccated pod, containing mature seed, eventually shatters.

Molecular studies of the penultimate stage of pod development have revealed a spatial and temporal correlation between the up-regulation of a number of mRNAs and pod dehiscence in *B. napus.* These mRNAs encode a polygalacturonase (PG) and a proline-rich protein (SAC51). Further analysis of the expression of the PG following fusion of a pod-specific *Arabidopsis thaliana* PG promoter to GUS [Jenkins et al., (1997)], has revealed that reporter gene expression is restricted precisely to the layer of cells comprising the pod DZ in transgenic *B. napus.* From 40 DAA, Meakin and Roberts (1990b) reported a progressive increase in β-1,4-glucanase (cellulase) activity in the DZ.

It is understood that the processes of dehiscence and abscission are not regulated by the same environmental or chemical signals, but that they involve controlled degradation of cell wall material and cell separation in a distinct group of cells. Both ethylene and indole-3-acetic acid (IAA) appear to be important regulators of the timing of the abscission process but the role of these plant hormones in dehiscence is less clearly defined. The increase in cellulase activity has been shown to correlate with a rise in the production of ethylene, mainly from the seed, which peaks at around 40 DAA [Meakin and Roberts, 1990b; Johnson-Flanagan and Spencer, 1994].

Developmental processes, such as pod dehiscence, which involve highly regulated and controlled expression of an array of different genes at a precise time and cellular location, clearly require an intricate signal transduction network.

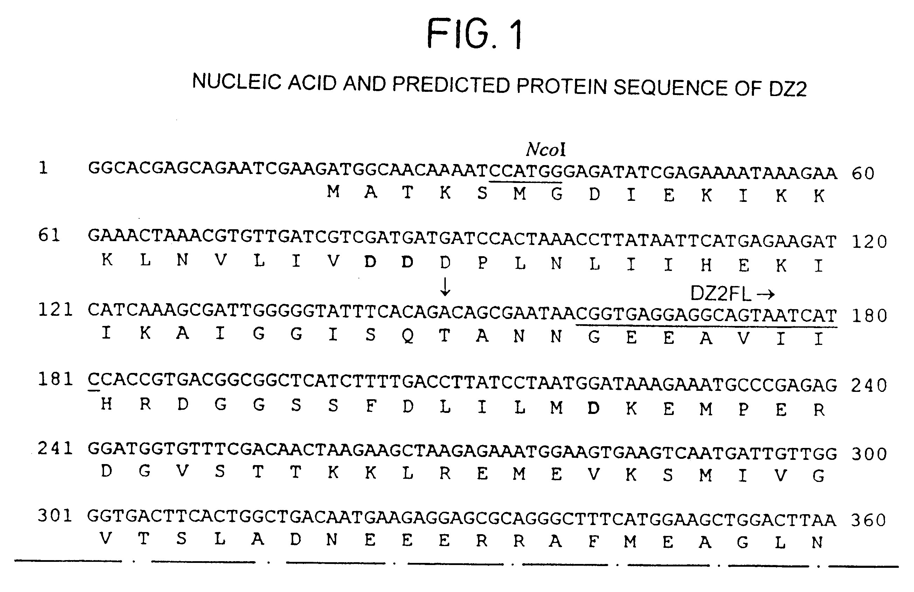

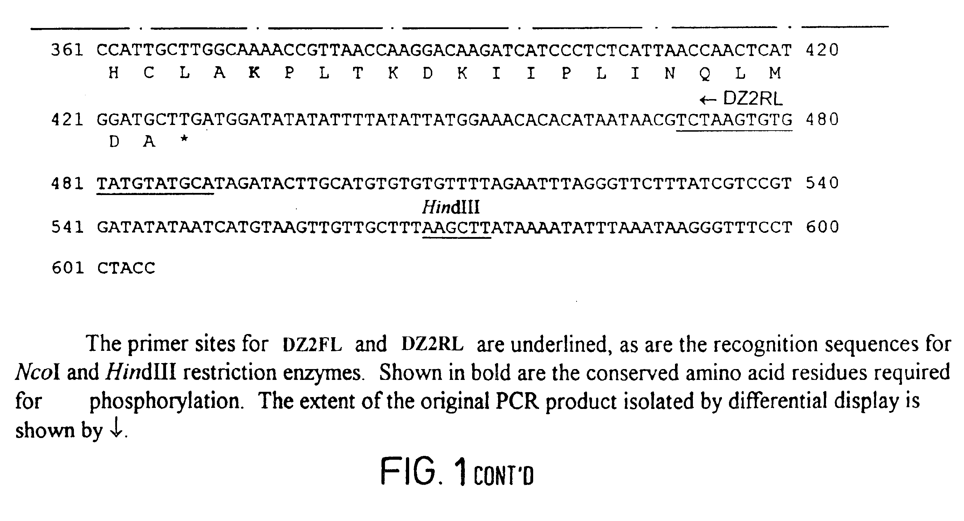

Further and improved genetic elements to control plant processes in this area are constantly desired. We describe the isolation, for the first time, of a plant cDNA (DZ2) encoding an individual response regulator protein, the expression of which is closely correlated with dehiscence of fruit in *B. napus.* DZ2 has a role in the ability to control molecule signaling during the events leading to shatter and thus to control pod shatter in plants. In addition to the identification of the nucleic acid termed "DZ.2" a homologous, but not identical sequence and protein were also identified from *B. napus.* This sequence was designated "DZ2B". Sequence analysis of DZ2 and DZ2B shows that there are two DZ2 genes in *B*. *napus,* each represented by a slightly different cDNA (here termed DZ2 and DZ2B). This is consistent with one gene being encoded by the *B. campestris* derived genome and the other from the genome derived from *B. oleracea.* In this text, the designation ."DZ2" is equivalent to the CW1 designation in UK 9806113.8 (as seen from Figure 1).

According to a first aspect of the invention there is provided nucleic acid optionally encoding a signal transduction protein involved in the process of dehiscence as described herein. Such a sequence or signal transduction protein has never previously been described in plant dehiscence.

In this text, the term "involved in the process of dehiscence" means any nucleic acid (preferably) encoding any protein which has an effect in the dehiscence process, in particular a protein or nucleic acid sequence involved in an MAP Kinase cascade or any other protein or nucleic acid sequence which results in changes in the expression of genes involved in dehiscence, such as upregulation of genes encoding polygalacturonase, cellulase, senescence-related proteins and/or downregulation of genes encoding for proteins involved in cells wall biosynthesis. The nucleic acid sequences/proteins of the present invention which are " involved in the process of plant dehiscence" are not the individual structural genes or proteins which cause dehiscence (polygalacturonases etc.). Rather, the nucleic acid sequences/proteins of the present invention are sequences/proteins which have an effect on the expression of such structural genes or proteins. One advantage of the present invention is that the use of such nucleic acid sequences/proteins enables the possibility to influence the whole process of dehiscence rather than just one element of it. Preferably the protein or nucleic acid sequence of the present invention which is involved in the process of dehiscence effects a structural protein which is a hydrolytic enzyme such as polygalacturonase or cellulase.

The nucleic acid of the first aspect of the invention may be a nucleic acid which is naturally expressed in a dehiscence zone. Such a nucleic acid will most accurately reflect nucleic acid naturally expressed in a plant. Preferably the dehiscence zone is a pod (also termed "siliques), anther and/or funiculus dehiscence zone. Preferably the plant is a member of the Brassica family, maize, wheat, soyabean, *Cuphea* or sesame.

A second aspect of the invention provides nucleic acid encoding a protein wherein the protein:
a) comprises the amino acid sequence shown in figure 1 or:
b) has one or more amino acid deletions, insertions or substitutions relative to a protein as defined in a) above, but has at least 95% amino acid sequence identical therewith,
   wherein said protein is involved in dehiscence.

The percentage amino acid identity can be determined using the default parameters of the GAP computer program, version 6.0 described by Deveraux *et al.,* 1984 and available from the University of Wisconsin Genetics Computer Group (UWGCG). The GAP program utilises the alignment method of Needleman and Wunsch 1970 as revised by Smith and Waterman 1981. More preferably the protein has 95% identity to the amino acid sequence of Figure 1, using the default parameters.

The skilled person will appreciate that various changes can sometimes be made to the amino acid sequence of a protein (which has a desired property) to produce variants (often known as "muteins") which still have said property. Such variants of the protein describe in a, b and c above are within the scope of the present invention and are discussed in greater detail below in sections *(i)* to *(iii).* They include allelic and non-allelic variants.

### (i) Substitutions

An example of a variant of the present invention is a polypeptide as defined in a, b or c above, apart from the substitution of one or more amino acids with one or more other amino acids.

The skilled person is aware that various amino acids have similar characteristics. One or more such amino acids of a protein can often be substituted by one or more other such amino acids without eliminating a desired property of that protein.

For example, the amino acids glycine, alanine, valine, leucine and isoleucine can often be substituted for one another (amino acids having aliphatic side chains). Of these possible substitutions it is preferred that glycine and alanine are used to substitute for one another (since they have relatively short side chains) and that valine, leucine and isoleucine are used to substitute for one another (since they have larger aliphatic side chains which are hydrophobic). Other amino acids that can often be substituted for one another include phenylalanine, tyrosine and tryptophan (amino acids having aromatic side chains); lysine, arginine and histidine (amino acids having basic side chains); aspartate and glutamate (amino acids having acidic side chains); asparagine and glutamine (amino acids having amide side chains); and cysteine and methionine (amino acids having sulphur containing side chains).

Substitutions of this nature are often referred to as "conservative" or "semi-conservative" amino acid substitutions.

### (ii) Deletions

Amino acid deletions can be advantageous since the overall length and the molecular weight of a polypeptide can be reduced whilst still retaining a desired property. This can enable the amount of protein required for a particular purpose to be reduced. Proteins according to the present invention, which have such deletion(s) are useful. They may interfere with the normal functioning of DZ2; that is, they may act as dominant negative mutations preventing normal DZ2 functioning and thus be of particular value, for example, in reducing pod shatter.

The amino acid sequence shown in figure 1 has various functional regions. For particular applications of the present invention, one or more of these regions may not be needed and may therefore be deleted.

### (iii) Insertions

Amino acid insertions relative to a polypeptide as defined in a, b or c above can also be made. This may be done to alter the nature of the protein (e.g. to assist in identification, purification, or expression, as explained below in relation to fusion proteins).

Changes in the protein according to the present invention can produce versions of the protein that are constitutively active. If a protein of the present invention acts on an inhibitor of the release of hydrolytic enzymes, then a constitutively active version would prevent or reduce pod shatter

A protein according to any aspect of the invention may have additional N-terminal and/or C-terminal amino acid sequences. Such sequences can be provided for various reasons. Techniques for providing such sequences are well known in the art. They include using gene-cloning techniques to ligate together nucleic acid molecules encoding polypeptides or parts thereof, followed by expressing a polypeptide encoded by the nucleic acid molecule produced by ligation.

Additional sequences may be provided in order to alter the characteristics of a particular polypeptide. This can be useful in improving expression or regulation of expression in particular expression systems. For example, an additional sequence may provide some protection against proteolytic cleavage. This has been done for the hormone somatostatin by fusing it at its N-terminus to part of the β galactosidase enzyme [Itakwa *et al.,* 105-63 (1977)].

Additional sequences can also be useful in altering the properties of a polypeptide to aid in identification or purification.

For example, a signal sequence may be present to direct the transport of the polypeptide to a particular location within a cell or to export the polypeptide from the cell. Hydrophobic sequences may be provided to anchor a polypeptide in a membrane. Thus the present invention includes within its scope both soluble and membrane-bound polypeptides.

Preferably, the nucleic acid according to the second aspect of the invention encodes a signal transduction protein or a functional portion thereof involved in the process of dehiscence. All preferred features of the first aspect of the invention as described above also apply to the second.

The term protein used in this text means, in general terms, a plurality of amino acid residues joined together by peptide bonds. It is used interchangeably and means the same as polypeptide or peptide.

The nucleic acid according to the invention comprises the sequence set out in figure 1 or a sequence which is 95% identical, to the sequence in Figure 1 at the nucleic acid residue level, using the default parameters of the GAP computer program, version 6.0 described by Deveraux *et al.,* 1984 and available from the University of Wisconsin Genetics Computer Group (UWGCG). The GAP program utilises the alignment method of Needleman and Wunsch 1970 as revised by Smith and Waterman 1981. While this nucleic acid is the preferred nucleic acid of the invention, it is well known to those persons skilled in the art that because of the nucleic acid "degenerate code" which encodes nucleic acids, a considerable number of variations in nucleic acid sequence can be used to encode for proteins according to the first or second aspects of the invention.

The nucleic acid of the invention may be isolated or recombinant and may be in substantially pure form. The nucleic acid may be antisense to nucleic acid according to the first or second aspects of the invention. As understood by the person skilled in the art introducing the coding region of a gene in the reverse orientation to that found in nature (antisense) can result in the downregulation of the gene and hence the production of less or none of the gene product. The transcribed antisense DNA is capable of binding to and destroying the function of the sense RNA of the sequence normally found in the cell, thereby disrupting function. Antisense nucleic acid may be constitutively expressed, but is preferably limited to expression in those zones (dehiscence) in which the naturally occurring nucleic acid is expressed.

The nucleic acid according to the invention preferably include a promoter or other regulatory sequence which controls expression of the nucleic acid. Promoters and other regulatory sequences which control expression of a nucleic acid in dehiscence zones are known in the art, for example described in WO96/30529 and WO94/23043. Further promoters or other regulatory sequences can be identified and can also include the promoter or other regulatory sequence which controls expression of a nucleic acid as set out in figure 1. The person skilled in the art will know that it may not be necessary to utilize the whole promoter or other regulatory sequence. Only the minimum essential regulatory elements may be required and in fact such elements can be used to construct chimeric sequences or promoters. The essential requirement is, of course, to retain the tissue and/or temporal specificity.

The nucleic acid according to the invention may be in the form of a vector. The vector may be a plasmid, cosmid or phage. Vectors frequently include one or more expressed markers which enable selection of cells transfected (or transformed: the terms are used interchangeably in this text) with them and preferably, to enable a selection of cells containing vectors incorporating heterologous DNA. A suitable start and stop signal will generally be present and if the vector is intended for expression, sufficient regulatory sequences to drive expression will be present. Nucleic acid according to the first and second aspects of the invention is preferably for expression in plant cells and thus microbial host expression is perhaps less important although not ruled out. Microbial host expression and vectors not including regulatory sequences are useful as cloning vectors.

A third aspect of the invention relates to a cell transformed with nucleic acid according to the invention. The cell may be termed as "a host" which is useful for manipulation of the nucleic acid, including cloning. Alternatively, the cell may be a cell in which to obtain expression of the nucleic acid, most preferably a plant cell. The nucleic acid can be incorporated by standard techniques known in the art in to cells. Preferably nucleic acid is transformed in to plant cells using a disarmed Ti plasmid vector and carried by an Agrobacterium by procedures known in the art, for example as described in EP-A-0116718 and EP-A-0270822. Foreign nucleic acid can alternatively be introduced directly into plant cells using an electrical discharged apparatus or by any other method that provides for the stable incorporation of the nucleic acid into the cell. Preferably the stable incorporation of the nucleic acid is within the nucleic DNA of any cell preferably a plant cell. Nucleic acid according to the first and second aspects of the invention preferably contains a second "marker" gene that enables identification of the nucleic acid. This is most commonly used to distinguish the transformed plant cell containing the foreign nucleic acid from other plants cells that do not contain the foreign nucleic acid. Examples of such marker genes include antibiotic resistance, herbicide resistance and Glucuronidase (GUS) expression. Expression of the marker gene is preferably controlled by a second promoter which allows expression of the marker gene in cells other than those than dehiscence zones (if this is the tissue specific expression of the nucleic acid according to the first or second aspects of the invention). Preferably the cell is from any of the Brassica family (most preferably *B. napus),* maize, wheat, soyabean, *Cuphea* and sesame.

A third aspect of the invention includes a process for obtaining a cell comprising nucleic acid according to the invention. The process involves introducing said nucleic acid into a suitable cell and optionally growing on or culturing said cell.

A fourth aspect of the invention provides a plant or a part thereof comprising a cell according to the third aspect of the invention. A whole plant can be regenerated from the single transformed plant cell by procedures well known in the art. The invention also provides for propagating material or a seed comprising a cell according to the third aspect of the invention. The invention also relates to any plant or part thereof including propagating material or a seed derived from any aspect of the invention. The fourth aspect of the invention also includes a process for obtaining a plant or plant part (including propagating material or seed, the process comprising obtaining a cell according to the third aspect of the invention or, indeed, plant material according to the fourth aspect of the invention and growth (to the required plant, plant part, propagating material etc). Techniques for such a process are commonplace in the art.

A fifth aspect of the invention provides a signal transduction protein involved in the process of the plant dehiscence. The signal transduction protein according to the fifth aspect may have one or more of the preferred features according to the first or second aspects of the invention. Preferably it may be isolated, recombinant or in substantially pure form. It may comprise the various changes according to the first or second aspects. Preferably the protein is expressed from nucleic acid according to the first or second aspects. Alternatively, the protein can be provided using suitable techniques known in the art.

this aspect of the invention provides a protein which;
a) comprises the amino acid sequence shown in figure 1 or;
b) has one or more amino acid deletions, insertions, or substitutions relative to a protein as defined in a) above and has at least 95% amino acid sequence identity therewith,
   wherein said protein is involved in dehiscence.
The percentage amino acid identity can be determined using the default parameters of the GAP computer program, version 6.0 described by Deveraux *et al*., 1984 and available from the University of Wisconsin Genetics Computer Group (UWGCG). The GAP program utilises the alignment method of Needleman and Wunsch 1970 as revised by Smith and Waterman 1981. More preferably the protein has 95% identity to the amino acid sequence of Figure 1, using the default parameters.

The protein is preferably a signal transduction protein involved in the process of plant dehiscence.

The seventh aspect of the invention provides a process for obtaining a shatter-resistant Brassica napus plant or in a part thereof, the process comprising transforming a Brassica napus plant or a part thereof with a nucleic acid according to the fourth aspect of the invention. The process of dehiscence can be regulated and/or controlled by increasing or decreasing the expression of nucleic acid sequences according to the first or second aspect of the invention. Increased or decreased expression can easily be influenced by the person skilled in the art using technology well known. This includes increasing the numbers of copies of nucleic acid according to the invention in a plant or a plant thereof or increasing expression levels of copies of the nucleic acid present in particular parts or zones of the plant. Preferably the zones are dehiscence zones.

The process according to the seventh aspect of the invention includes obtaining a plant cell according to the third aspect of the invention or part of a plant according to the fourth aspect in the invention and deriving a plant therefrom. Alternatively, the process may comprise obtaining propagating material or a seed according to the fourth aspect of the invention and deriving a plant therefrom.

Preferably, the process of the seventh aspect of the invention is in the pod or the anther of a plant. All preferred features of aspects one to six also apply to the seventh.

An eighth aspect of the invention provides for the use of nucleic acid according to the first to seventh aspects of the invention in the regulation/control of plant dehiscence. All preferred features of aspects one to seven also applies to the eighth.

The ninth aspect of the invention makes it possible to use of nucleic acid according to the first or second aspect of the invention as a probe. Such a probe can be used in techniques well known in the art to identify the presence of identical or homologous nucleic acid sequences from any source, preferably a plant source. The ninth aspect of the invention also provides nucleic acid identified by use of the nucleic acid from aspects one or two as a probe.

A tenth aspect of the invention provides for the use of nucleic acid according to aspects one or two of the invention in the production of a cell, tissue, plant or part thereof, or propagating material. Again, all preferred features of aspects one and two also apply to the tenth.

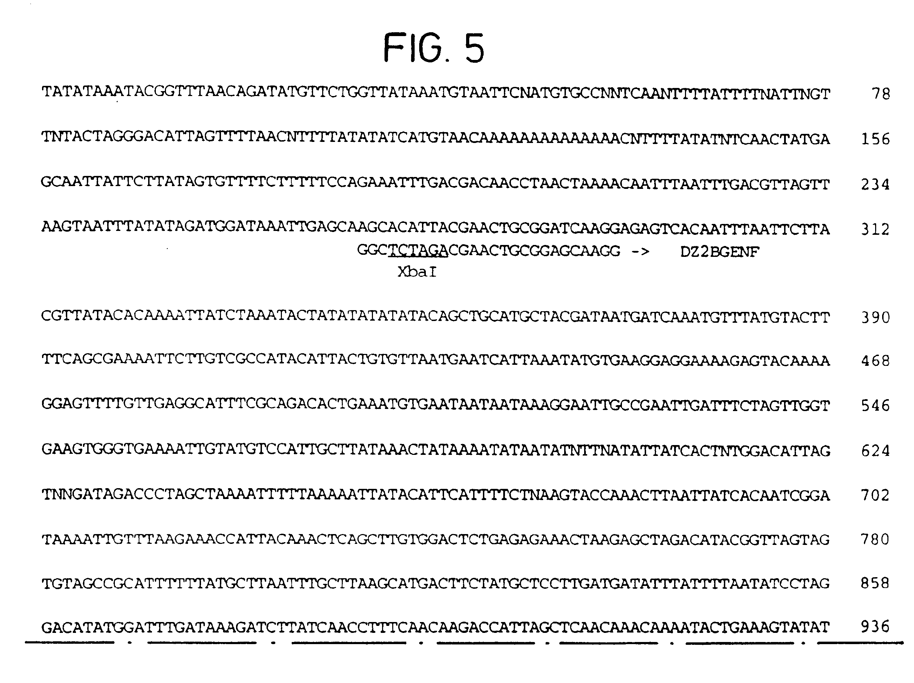

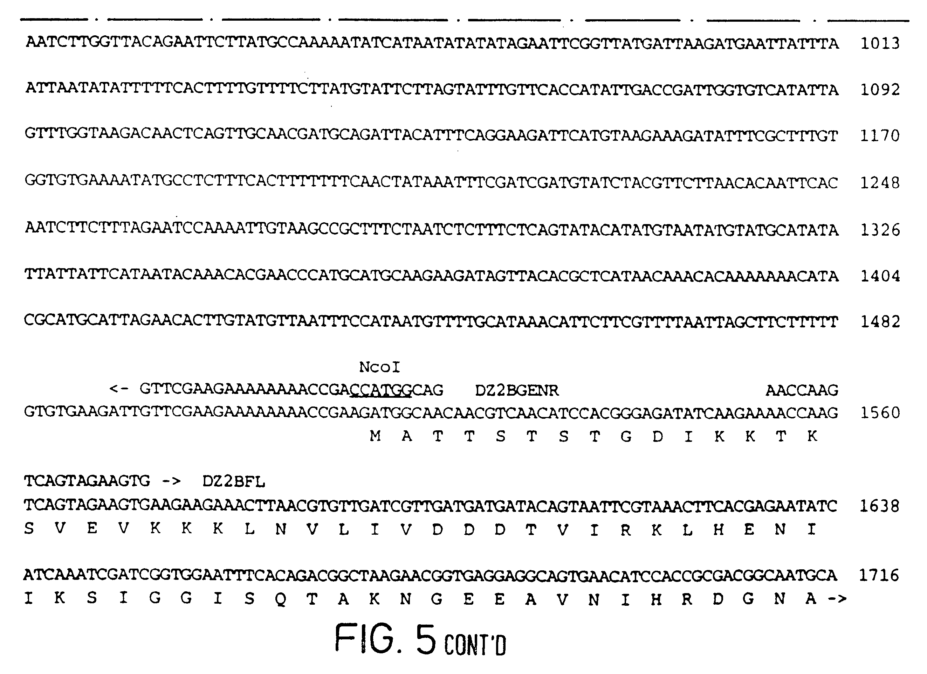

An eleventh aspect of the invention provides for nucleic acid comprising one or more of the underlined sequences as set out in figure 1 or the primer sequences in Fig 5, Fig 9 or Fig 11. Such nucleic acid sequences are preferably used as primers in an PCR (Polymerase Chain Reaction) process in order to amplify nucleic acid sequences.

A twelfth aspect of the invention makes it possible to use nucleic acid according to the first or second aspects of the invention to identify another other protein or proteins which interact with its expression product. Such use can be carried out by the yeast two hybrid screening method (or others known in the art). The yeast two hybrid screening method is described for this aspect of the invention, in general, with reference to the sequence described as DZ2. A potential way to implement the yeast 2-hybrid screen is outlined, as follows:

DZ2 is linked to the Gal4 DNA binding domain and expressed in yeast which contains a pGAL4-lacZ gene. For activity of lacZ a second protein is required that contains the DNA transcriptional activation domain of GAL4 and that interacts with the DZ2 protein. This is provided by making a cDNA expression library from plant DZ zones which results in fusions of plant proteins to the GAL4 activation domain. This library is transformed into the yeast strain that contains pGAL4-LacZ and expresses the DZ2-Ga14 DNA bining domain protein fusion. Colonies that have lacZ activity are transformed with a gene for a protein that interacts with DZ2.

Using such a system, upstream and downstream components of any signal transduction pathway can be identified, thus resulting in further ability to control/regulate dehiscence and/or male sterility.

A fourteenth aspect of the invention makes it possible to use a protein according to the fifth or sixth aspect of the invention as a probe. In this context the probe is a means to identifying interacting entities (such as other proteins), including upstream and downstream interacting signal components. A protein according to the fifth or sixth aspect of the invention can be used as a probe to directly look for interactions with other proteins, i.e. purified protein can be used to look for complex formation with other plant protein, particularly isolated from the DZ zone. For example, a modified recombinant DZ2 protein can be made with a sequence tag, such as a Histag, that enables the DZ2 + interacting protein to be directly purified on a His affinity column. Alternatively, an antibody can be raised to DZ2 protein. This antibody is then used to identify DZ2 protein complexes and to purify the complexes. The DZ2 interacting proteins can be purified and microsequenced to enable cloning of the genes for these interacting proteins.

The present invention provides a particularly useful method by which plant dehiscence can be regulated/controlled.

In addition to the use of the present invention in the production of shatter resistance or shatter-delayed plants such as oil seed rape, the invention may be used to control/regulate pollen release (by the control/regulation of anther dehiscence) which can produce male sterile plants. The temporal and spatial expression of nucleic acid encoding a protein according to the first and second aspects of the invention may require adjustment in obtaining the correct levels of dehiscence delay or prevention in different zones. For example, if pod dehiscence is required but anther dehiscence is not, it is necessary to ensure that expression of nucleic acid according to a first or second aspect of the invention has the correct temporal and spatial expression in order to obtain pod dehiscence or delay but not, to any substantial extent, anther dehiscence. This can be obtained by processes known in the art and may require use of particular promoter sequences to obtain the desired result. Usually in plant transformation, some difference in the level of expression of nucleic acid is observed in different plants. In some cases, the ratio of expression levels in different tissues can vary between different plant transformants thus providing essentially tissue-specific expression in one or other of the target tissues in some of the plant transformants. In the present invention, the natural expression of nucleic acid according to the first or second aspects may be predominantly higher in pod dehiscence zones and lower in the anther and funiculus dehiscence zones. However, as described above, it is possible to obtain plants in which the protein expression is regulated in a particular dehiscence zone. Accordingly, a particularly useful aspect of the invention is the provision of plants which have one or both of the following features; are male sterile, are shatter resistant.

As described earlier, the process of dehiscence at the dehiscence zone involves the secretion of a number of enzymes, including hydrolytic enzymes. While previous attempts have been made to down or up regulate specific genes encoding particular proteins involved in the process of dehiscence, regulation by means of a signal transduction protein which effects expression of a number of genes is likely to be more effective than regulation of a single gene. In addition to this, the nucleic acid of the present invention has been identified as being expressed earlier than several other known genes involved in the process of plant dehiscence. This suggests that it is important earlier on in the process of plant dehiscence and can be used to control/regulate plant dehiscence at an earlier stage.

The nucleic acid encoding a signal transduction protein involved in the process of dehiscence or the signal transduction protein itself may be a component of a signal pathway that may either positively or negatively regulate pod shatter.

A more detailed explanation of such regulations/control, described with reference to a pod shatter (dehiscence) model is described below. As a skilled person will acknowledge, the model described below also relates to other general processes of dehiscence such as in the anther.

In the process of dehiscence, a particular signal transduction protein may be required to transmit a signal from the almost mature seed which initiates the expression or release of enzymes required for pod shatter. In this model, developmental signals switch on expression and/or activation of a particular signal transduction protein in the pod dehiscence zone. This leads to expression of genes required for the release of pod dehiscence zone enzymes (such as hydrolytic enzymes). In this case, prevention of activity of the signal transduction protein, for example by downregulation of expression of this protein, would result in reduced dehiscence. Alternatively, the developing seed may transmit a signal which represses the expression and/or activity of a particular signal transduction protein until late in cell development. In this model, developmental signals switch on a particular signal transduction protein which, in due course, represses the expression of genes required for release of dehiscence zone specific enzymes (such as hydrolytic enzymes). In this case, expression of a modified signal transduction protein that is constitutively active would result in reduced dehiscence.

A signal transduction protein which is either positively or negatively involved in the process of dehiscence can be used according to the present invention.

In addition to DZ2 several other DZ-expressed genes have been previously isolated and individually downregulated to result in *B. napus* plants that have increased resistance to pod shatter; namely Sac66 (WO 96/30529 - Figure 15), DZ15 (Figure 16) and OSR 7(9) (Figure 17). It is anticipated that downregulation of more than one gene involved in pod shatter will further increase resistance to pod shatter. This could be achieved by combining different transgenes by transformation with several transgenes each designed to downregulate a different DZ-expressed gene or by crossing together *B. napus* lines that individually are transformed with such transgenes. Such methods are complex either involving transformation with a construct containing multiple chimeric genes or require the maintenance of several transgenic loci in the breeding program. A preferred method is to transform with a chimeric gene consisting of a single promoter driving expression of an antisense or partial sense transcript which is comprised of elements of all the DZ-expressed genes to downregulated. Similarly a single promoter could be used to drive the expression of multiple ribozymes each targeted against a different DZ-expressed gene. The use of a single promoter to drive expression of a combination of antisense, partial sense and ribozymes is also possible. Ideally the promoter will be pod DZ-specific, however a useful promoter may be pod-specific or even constitutively active. A suitable DZ-specific promoter would be that of DZ2. DZ2B, DZ2AT3 or ESJ2A (WO 99/13089).

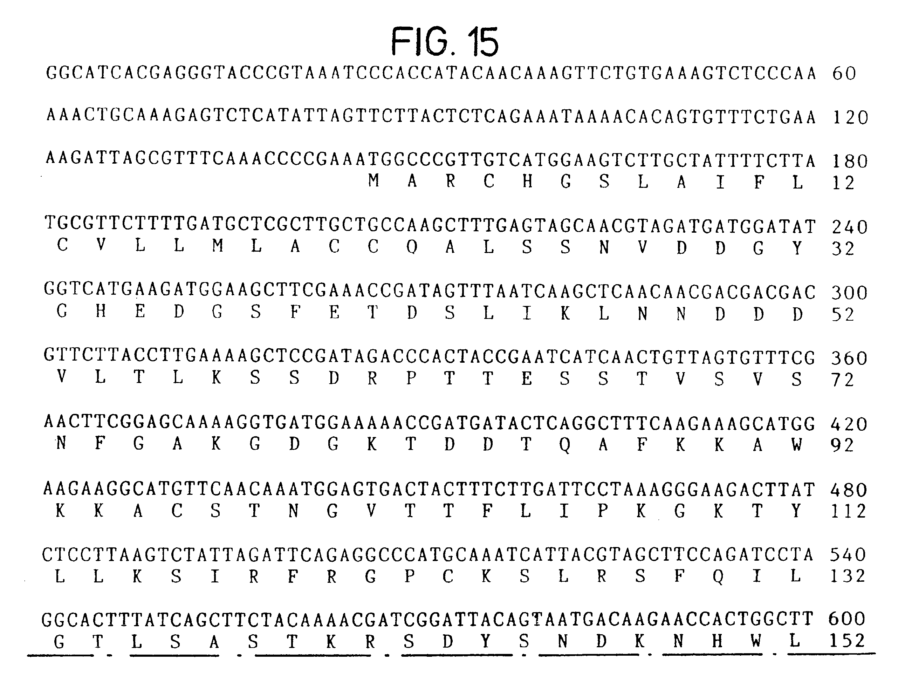

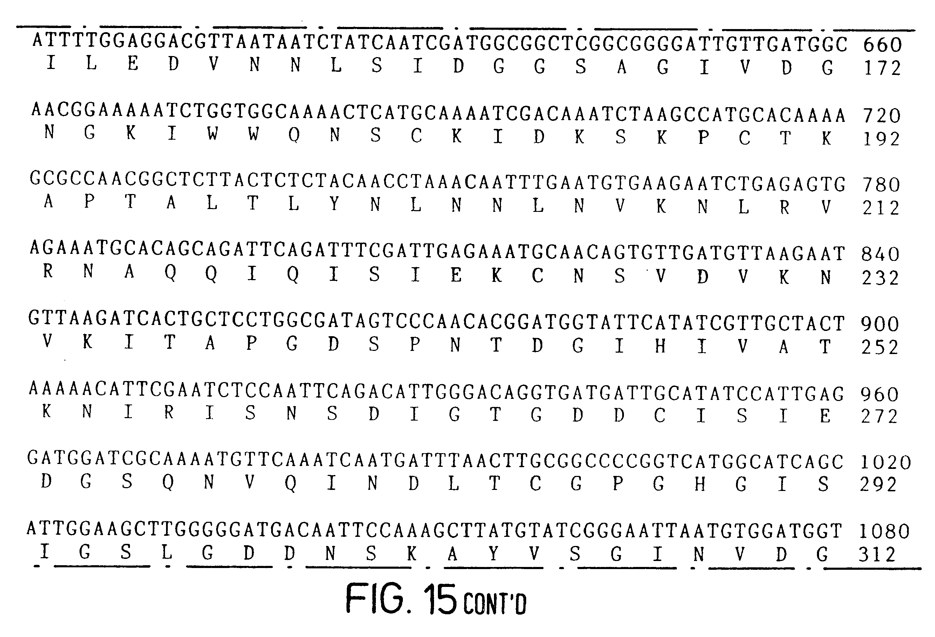

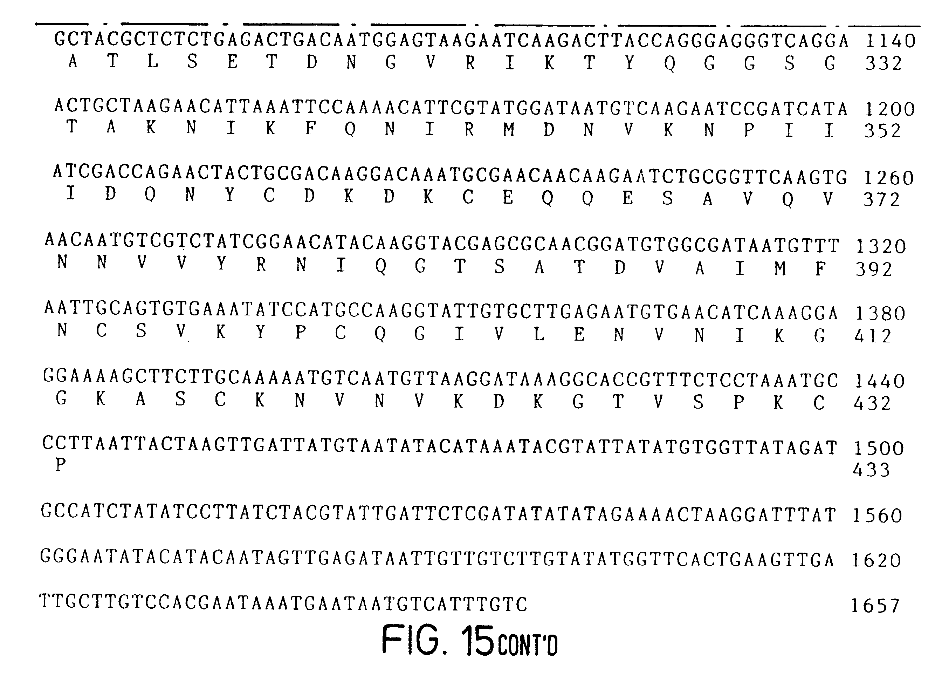

Accordingly, the present invention makes it possible to use a nucleic acid sequence according to the first or second aspects (and also all aspects which include the first or second aspects) in combination with one or more further nucleic acid sequences which are dehiscence-zone expressed. Examples of such sequences include Sac66, DZ15 and OSR(7), Figures 15-17 respectively. Such sequence may be in sense or in antisense orientation. Such a sequence may be included as full length genomic, full-length cDNA or partial sequences; the sequences may be as shown in the figures or may have the same sequence identity (both for aminoacid sequence and nucleic acid sequence) as described above for the protein according to the second aspect of the invention or the nucleic acid according to the first or second aspects of the invention. As will be recognised by those skilled in the art a partial sequence may be useful in either the sense or antisense orientation.

The invention is described by reference to the enclosed drawings;
- Figure 1: DZ2 full length sequence showing original PCR product and primer sites
- Figure 2: Amino acid alignment with bacterial response regulator proteins & *ETR1*
- Figure 3: Northern analysis of expression of DZ2 in pods and other tissues. The lower panel shows the ethidium bromide-stained RNA gel prior to blotting and probing with DZ2
- Figure 4: Comparison of bacterial two-component systems with DZ2
- Figure 5: Sequence of the promoter region of B.napus DZ2B.
- Figure 6: Nucleic and putative peptide sequence alignments of DZ2 with DZ2B.
- Figure 7: Northern analysis of expression of DZ2B in pods and other tissues. The probe was labeled DZ2B cDNA.
- Figure 8: Schematic diagram of pDZ2B-GUS-SCV
- Figure 9: DZ2AT3 cDNA sequence showing the putative DZ2AT3 peptide.
- Figure 10: Amino acid alignment of DZ2AT3 with DZ2 and DZ2B.
- Figure 11: Sequence of the promoter region of A.thaliana DZ2AT3.
- Figure 12: Schematic diagram of pDZ2AT3-GUS-SCV.
- Figure 13: Schematic diagram of pPGL-DZ2as-SCV and pDZ2B-DZ2as-SCV.
- Figure 14: Schematic diagram of pWP357-SCV.
- Table 1: Pod shatter resistance of WP357-SCV transformants.
- Figure 15: Nucleic acid sequence and putative amino acid sequence of Sac66.
- Figure 16: Nucleic acid sequence and putative amino acid sequence of DZ15.
- Figure 17: Nucleic acid sequence and putative amino acid sequence of OSR7 (9)

The present invention is now described with reference to the following, non-limiting examples.

### Example 1- Isolation and characterisation of expression of DZ2

### Plant Material

Seeds of *B. napus* cv Rafal were grown as described by Meakin and Roberts, (1990a) with the following modifications. Single seedlings were potted into 10cm pots, and after vernalization, were re-potted into 21cm pots. At anthesis tags were applied daily to record flower opening. This procedure facilitated accurate age determination of each pod. Pods were harvested at various days after anthesis (DAA). The dehiscence zone was excised from the non-zone material and seed using a scalpel blade (Meakin and Roberts (1990b)) and immediately frozen in liquid N₂ and stored at -70°C.

### RNA Isolation

All chemicals were molecular biology grade and bought from either Sigma Chemical Ltd (Dorset, UK), or Fisons (Loughborough, UK). Total RNA was extracted using the polysomal extraction method of Christoffersen and Laties, (1982), with the following alterations. The plant material was ground to a powder in liquid N₂ and then in 10 volumes of extraction buffer (200mM Tris-acetate [pH 8.2], 200mM magnesium acetate, 20mM potassium acetate, 20mM EDTA, 5% w/v sucrose, after sterilisation 2-mercaptoethanol was added to 15mM and cycloheximide added to a final concentration of 0.1 mg ml⁻¹). The supernatant was then layered over 8 ml 1M sucrose made with extraction buffer and centrifuged in a KONTRON^{™} (Switzerland) TFT 70.38 rotor at 45.000rpm (150,000g) for 2 hr at 2°C in a Kontron CENMKON^{™} T-1065 ultracentrifuge. Pellets were then resuspended in 500µl 0.1M sodium acetate, 0.1 % SDS, pH 6.0 and phenol/chloroform (1:1 v/v) extracted and the total RNA precipitated. Poly(A)⁺ RNA was isolated from total RNA extracted, from both the zone and non-zone tissue of 40, 45 and 50 DAA pods, using a Poly(A) QUIK^{™} mRNA purification kit (Stratagene, Cambridge, UK) following the manufacturers instructions, and then bulked together. Total RNA was also extracted from leaves, stems, seeds and pods using a method described by Dean *et al,* (1985) for use in Northern analyses.

### Differential display

This was performed essentially as described by Liang and Pardee (1992) using RNA extracted from 40 DAA pod dehiscence zones and non-zones. First strand cDNA copies of the RNAs (40 DAA DZ/NZ) were made using 50U M-MLV (Moloney Murine Leukemia Virus) reverse transcriptase (50U/µL) (Stratagene) in a 20µL reaction containing 1x M-MLV buffer, 2.5mM dNTPs (Pharmacia), 1µg RNA, 30U RNAse inhibitor (Promega) and 10µM oligo dT anchor primer 7 (5'-TTTTTTTTTTTTGG-3'). The reaction conditions were as follows: 65°C for 5 minutes, 37°C for 90 minutes and 95°C for 5 minutes. Following first strand cDNA synthesis, 60µL dH2O were added and the samples were either used directly for PCR or stored at -20°C.

For PCR, 2µL cDNA were used as template in a 20µL reaction containing 1x PCR buffer, 1mM MgCl₂, 2µM dNTPs, 10µM oligo dT anchor primer 7 (5'-TTTTTTTTTTTTGG-3'), 2.5µM arbitrary primer A (5'- AGC CAG CGA A -3'), 0.5µL 35S-dATP (> 1000 Ci/mmol) (Amersham) and 1U *Taq* DNA polymerase (5U/µL) (Gibco BRL). The thermocycling conditions were as follows: 40 cycles of 94°C for 30 seconds, 40°C for 2 minutes. 72°C for 30 seconds followed by 72°C for 5 minutes. The PCR products were fractionated on a 5% polyacrylamide/7M urea gel after addition of 5µL loading buffer (95% (v/v) formamide, 20mM EDTA, 0.05 % (w/v) xylene cyanol, 0.05% (w/v) bromophenol blue) to each sample. Following electrophoresis the gel was dried at 80°C under vacuum for 1 hour then exposed to X-ray film (BioMax-MR, Kodak) in a light tight cassette for 48 hours. The dried gel and autoradiogram were aligned so that bands that appeared in the DZ and not in NZ could be cut out and the DNA eluted according to Liang et al. (1995). The eluted PCR products (4µL) were reamplified in a 40µL reaction containing 1x PCR buffer, 1mM MgCl₂, 20µM dNTPs, 10µM oligo dT anchor primer 7 (5'-TTTTTTTTTTTTGG-3'), 2.5µM arbitrary primer A (5'- AGC CAG CGA A -3') and 2U *Taq* DNA polymerase (5U/µL) (Gibco BRL) using the following thermocycling conditions: 40 cycles of 94°C for 30 seconds, 40°C for 2 minutes, 72°C for 30 seconds followed by 72°C for 5 minutes. The resulting PCR product was cloned into the TA cloning vector (Invitrogen) and sequenced (Figure 1). In order to prepare an antisense strand-specific riboprobe, the PCR product was subcloned into pBluescript (Stratagene).

### Expression analysis and characterisation of DZ2

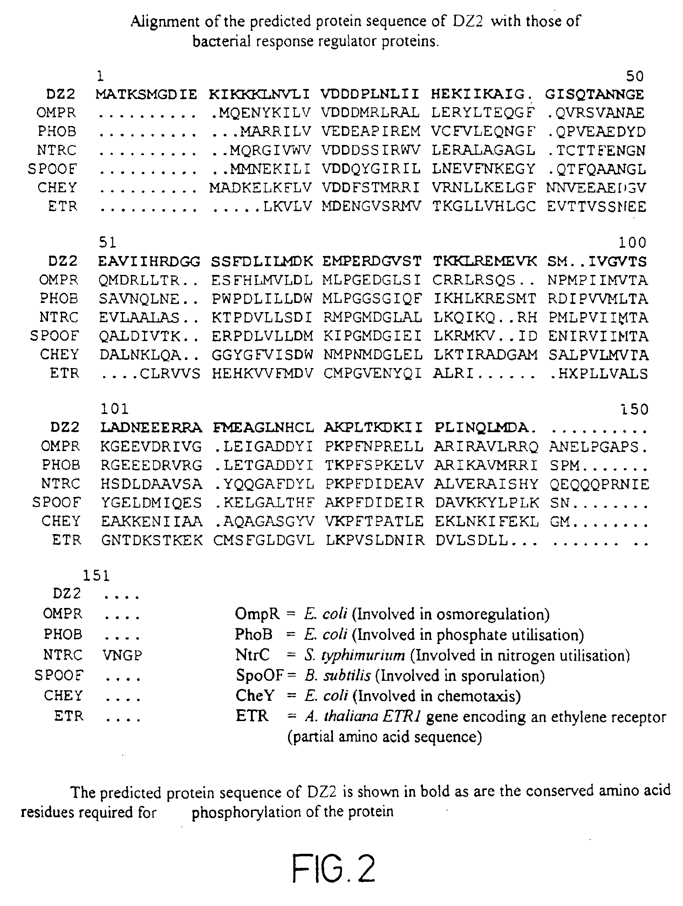

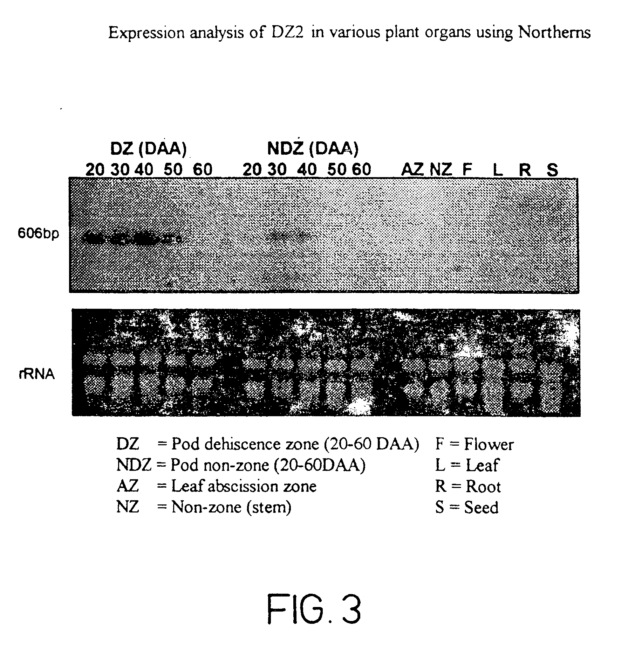

Northern analysis using an antisense strand-specific riboprobe to the DZ2 PCR product, showed that DZ2 hybridised to a transcript of 0.6kb which is expressed in the DZ of 20-50 DAA pods with a peak in expression at 40DAA. Minimal expression was observed in the pod NZ [Figure 2]. A random-primed labelled DNA probe (Stratagene) of the 330bp DZ2 PCR product (amplified using primers DZ2FL and DZ2RL - see Figure 1) was used to screen a *B. napus* DZ cDNA library from which, following three rounds of screening to obtain pure plaques, a full length DZ2 cDNA (606bp) was obtained (Figure 1). An antisense strand-specific riboprobe of the full length DZ2 cDNA was hybridised to total DNA extracted from pod DZ/NZ (as in Figure 2), leaf abscission zones (AZ) and non-zones (NZ) (following exposure to 10µL/L ethylene for 72 hours), seed, root, flower and leaf. Figure 3 shows that DZ2 hybridises to a 0.6kb message which is present in the pod DZ at 20-50 DAA with maximum expression at 40DAA. Again there is minimal expression in pod NZ and no apparent expression of DZ2 in AZ, NZ, leaf, root, seed or flower RNA. By the sensitive technique of RT-PCR analysis DZ2 expression can also be detected in anthers and the funiculus, both tissues that contain dehiscent zones

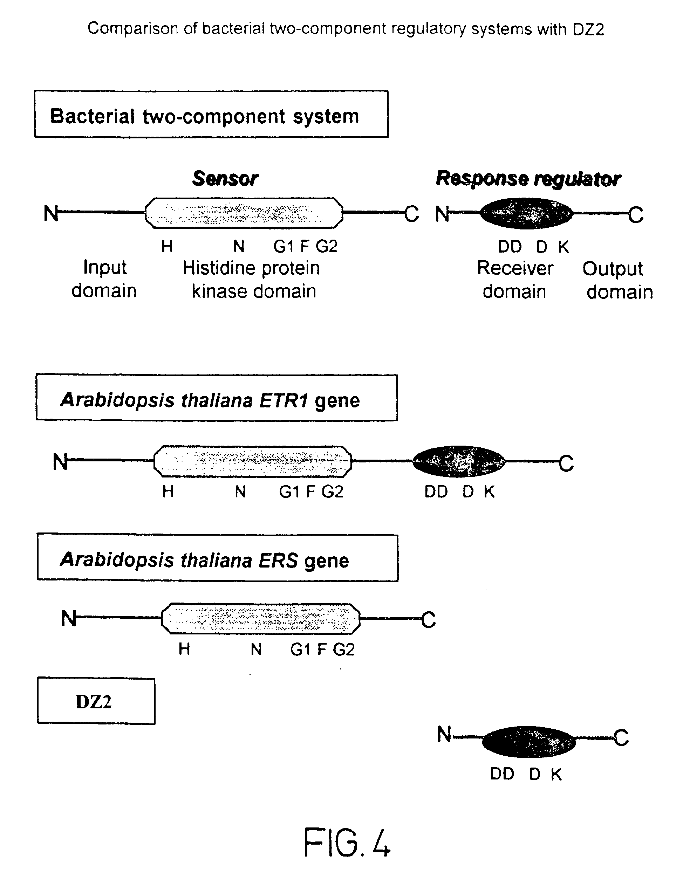

The 606bp cDNA (DZ2) encodes a putative protein of 136 amino acids. Comparison of the DZ2 translated sequence to the OWL protein database [Bleasby and Attwood (1994)] showed low but consistent homology to a group of bacterial proteins comprising two-component regulatory systems. In particular, DZ2 possesses the conserved amino acid residues required for phosphorylation of the receiver domain of the response regulator component (see Figure 4). DZ2 plays a role in a signal transduction cascade resulting at least in one respect in pod shatter. It is therefore a good candidate for down-regulation of pod shatter processes using antisense technology. DZ2 is a novel plant protein in that independent proteins with homology to bacterial receivers are yet to be reported in plants.

The full length cDNA was excised from the pBluescript cloning vector by digestion with *Eco*RI and *Xho*I restriction enzymes (Gibco BRL). Following purification from a 1 % agarose gel the 606bp cDNA was random primed labelled (Stratagene) and used to screen a *B. napus* genomic library in the BlueStar vector. Following three rounds of screening to obtain pure plaques, a single genomic clone was isolated which carries a 15kb genomic DNA insert. The promoter of the DZ2 gene is isolated from this genomic clone using standard techniques (see Example 2).

### Example 2 - Isolation and characterisation of the B.napus DZ2B promoter.

To obtain the B. napus DZ2 promoter a B.napus genomic library was screened with a labelled DZ2 probe. The full length cDNA was excised from the pBluescript cloning vector (Stratagene) by digestion with *Eco*RI and *Xh*oI restriction enzymes (Gibco BRL). Following purification from a 1% agarose gel the 606bp cDNA was random primed labelled (Stratagene) and used to screen a *B. napus* genomic library in the BlueStar vector (Novagen). Following three rounds of screening to obtain pure plaques, a single genomic clone was isolated which carries a 15kb genomic DNA insert. The region hybridising to DZ2 was sequenced and found to encode a protein homologous to, but not identical to DZ2. This DZ2-like gene was designated DZ2B (Figure 5). The primers DZ2BFL (Figure 5) and T7 were used to PCR out a DZ2B cDNA from the B.napus DZ cDNA library.

| | |
|---|---|
| 5'AACCAAGTCAGTAGAAGTG 3' | DZ2BFL |
| 5' AATACGACTCACTATAGG 3' | T7 |

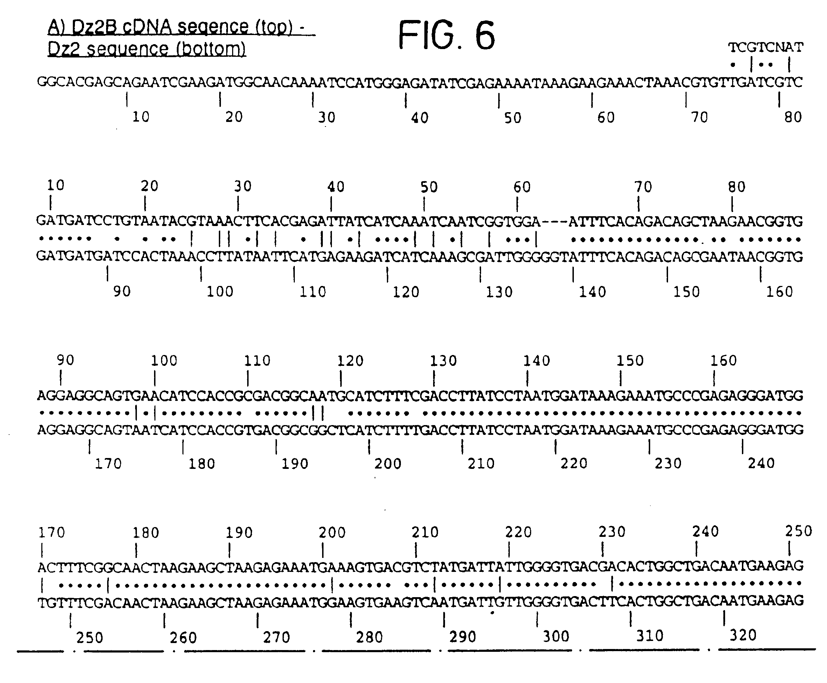

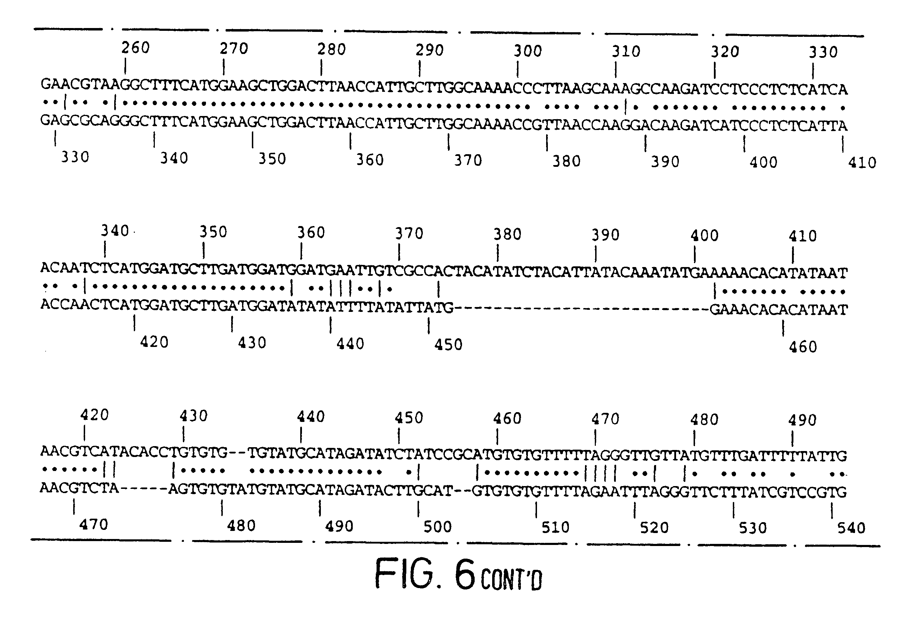

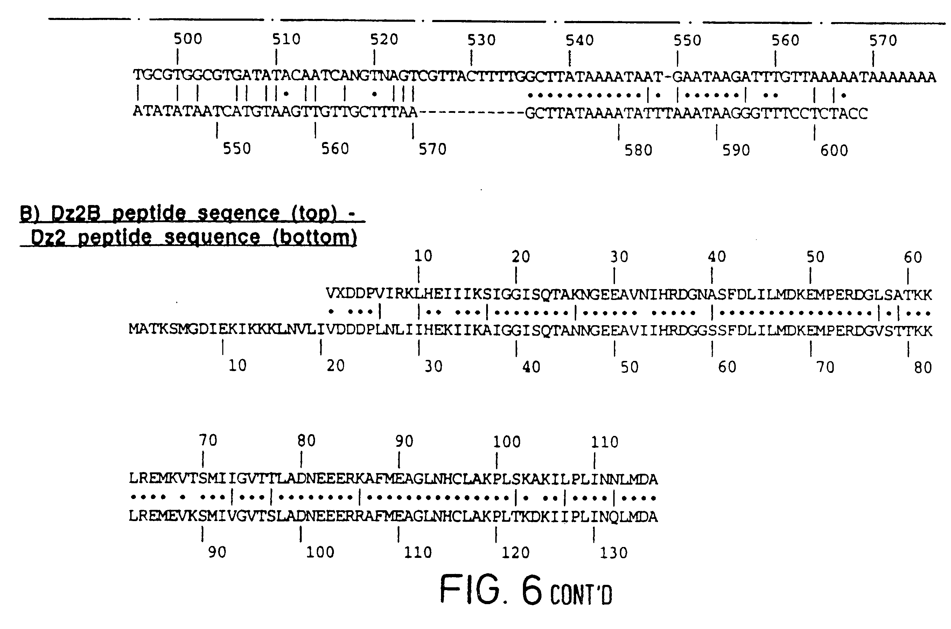

The DZ2 and DZ2B cDNAs are 80% identical (according to the default parameters of the GAP computer program, version 6, Deveraux *et al.,* 1984, and available from the University of Winsconsin Genetics Computer Group (UWGCG)) at the nucleotide level in the region of overlap of the coding sequences (Figure 6a) and the putative proteins encoded by DZ2 and DZ2B are 80% identical (according to the default parameters of the GAP computer program, version 6, Deveraux *et al.,* 1984, and available from the University of Winsconsin Genetics Computer Group (UWGCG)) (Figure 6b). Sequence analysis of more DZ2 and DZ2-like cDNAs and Southern analysis shows that there are two DZ2 genes in B.napus, DZ2 and DZ2B, each represented by 2 slightly different cDNAs. This is consistent with one gene being encoded by the B.campestris derived-genome and the other from the genome derived from B.oleracea.

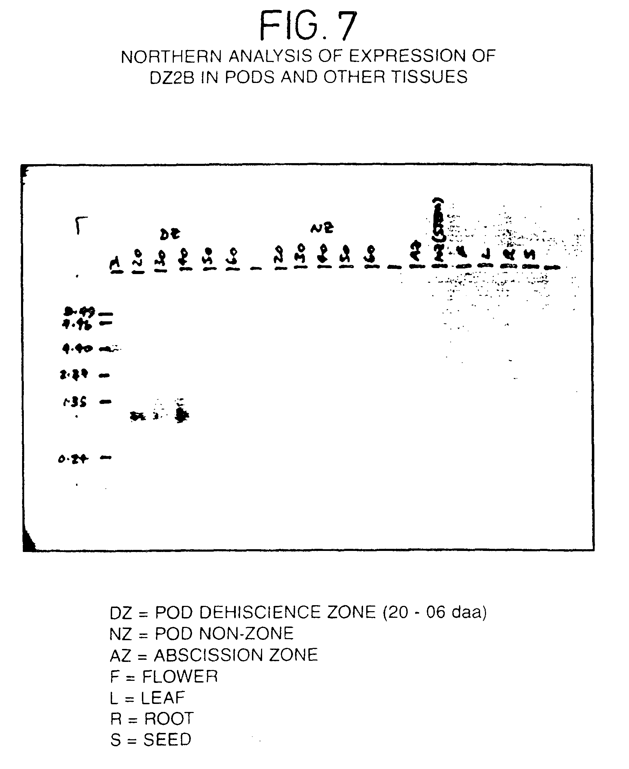

RT-PCR with primers specific to DZ2B showed that DZ2B is only expressed in pods. This was confirmed by northern analysis which showed preferential expression in the DZ (Figure 7). Thus DZ2B has a similar pattern of expression as DZ2 and is thus a suitable source of a DZ-expressed promoter.

Primers DZ2BGenF and DZ2BGenR were used to PCR a 1253bp DZ2B promoter fragment (Figure 5).

| | |
|---|---|
| 5' GGCTCTAGACGAACTGCGGAGCAAGG 3' | DZ2BGENF |
| 5' CTGCCATGGTCGGTTTTTTTTCTTCGAAC 3' | DZ2BGENR |

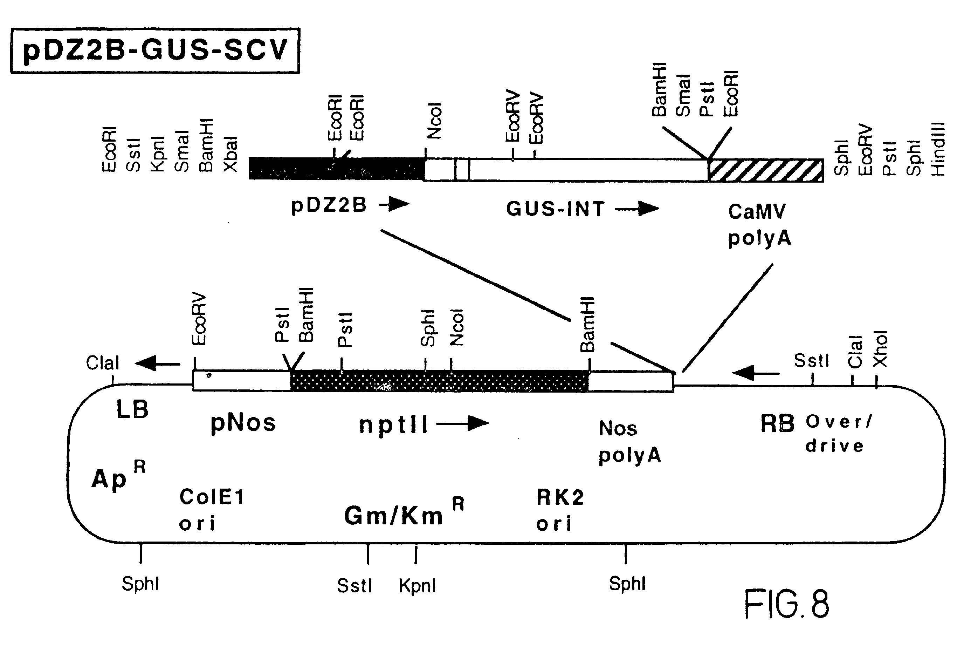

These primers introduced an XbaI site at the 5' end of the PCR fragment and an NcoI site around the initiating Met of DZ2B. Thus the PCR fragment was cloned as an XbaI, NcoI fragment between the XbaI and NcoI sites of pWP272 (WO 99/10389) forming pDZ2B-GUS. The chimeric pDZ2B-GUS-CaMV poly A gene was then transferred as an XbaI, XhoI fragment between the XbaI and SaII sites of pSCV nos-nptII (WO 95/20668) forming pDZ2B-GUS-SCV (Figure 8). The pDZ2B-GUS-SCV binary vector was transferred to the agrobacterial strain pGV2260 and transformed B.napus plants produced by agrobacterial transformation essentially as described in Moloney M et al., (1989). Gus expression is observed in the pod DZ.

### Example 3 - Isolation and characterisation of a DZ2 Arabidopsis thaliana homologue

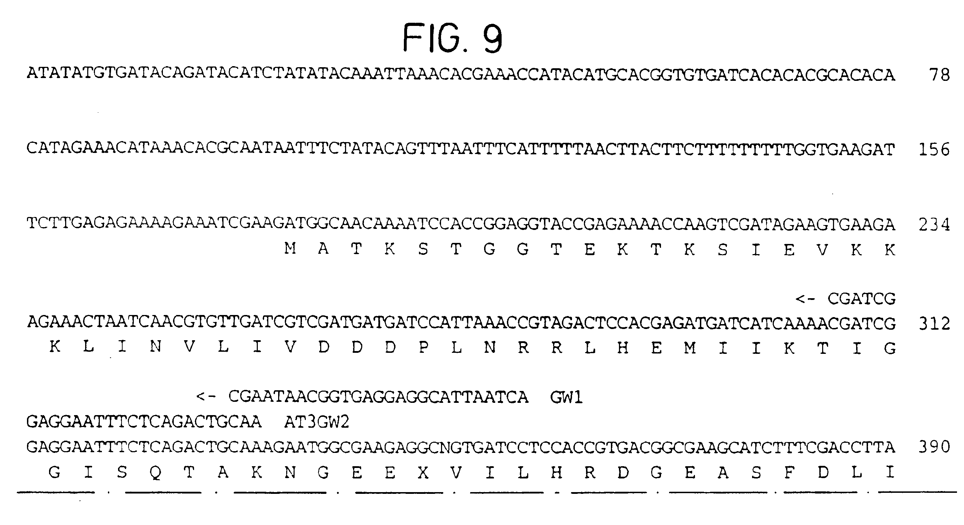

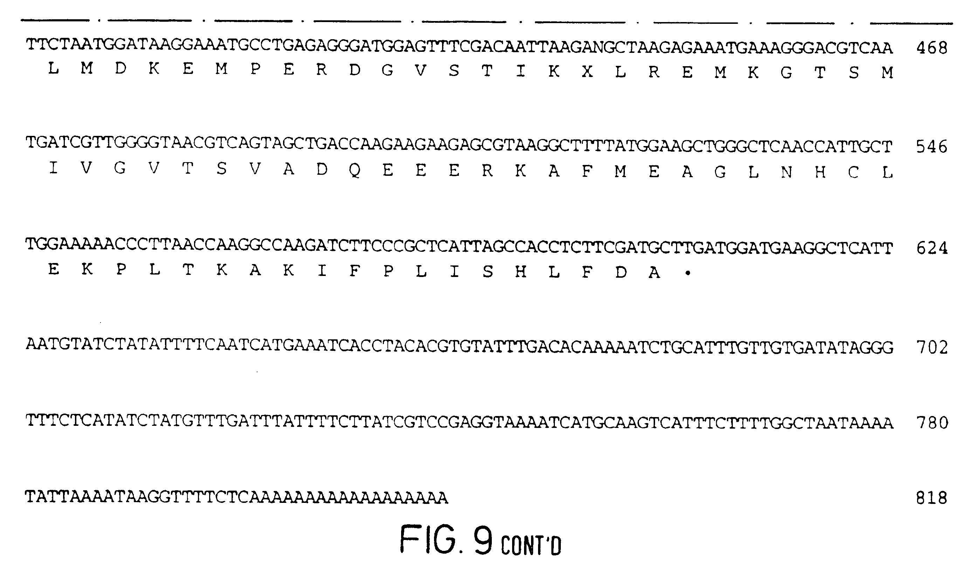

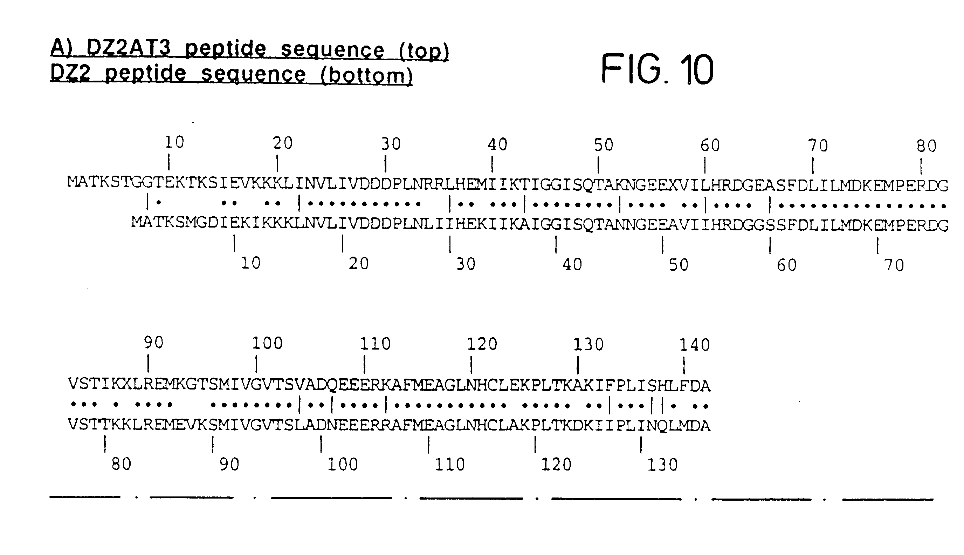

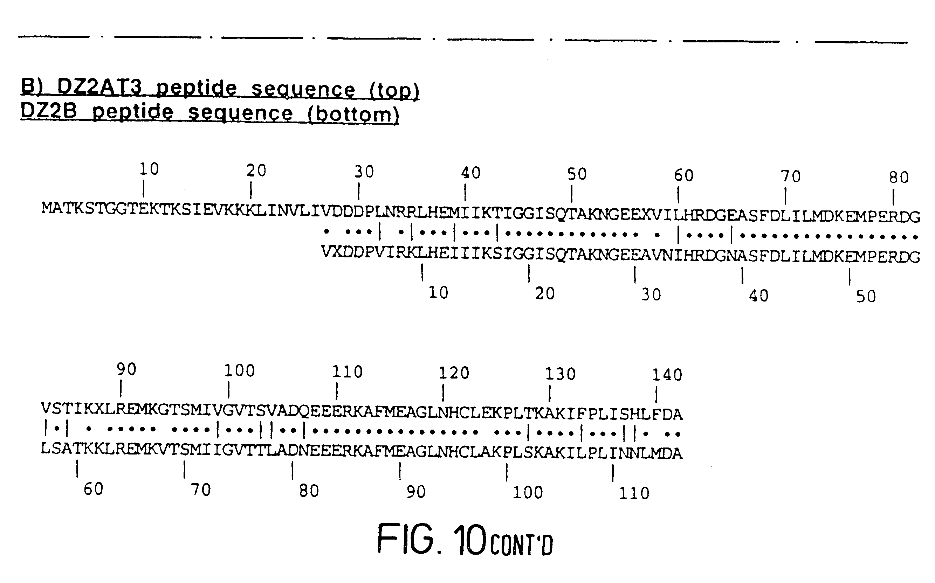

To demonstrate that a DZ2 orthologous gene can be isolated from another plant species the functional equivalent of B.napus DZ2 / DZ2B was isolated from Arabidopsis thaliana. The B.napus DZ2 cDNA was used as a probe to screen an Arabidopsis cDNA library (J. Giraudat, ISV-CNRS, France). Figure 9 shows the sequence of a cDNA (DZ2AT3) that hybridised to the DZ2 probe. DZ2AT3 has 85 % nucleic acid identity to DZ2 and 85% to DZ2B (according to the default parameters of the GAP computer program, version 6, Deveraux *et al.,* 1984, and available from the University of Winsconsin Genetics Computer Group (UWGCG)) in the coding regions which are common to all three sequences. The putative peptide encoded by DZ2AT3 has 80% identity to DZ2 and 80% to DZ2B (according to the default parameters of the GAP computer program, version 6, Deveraux *et al.,* 1984, and available from the University of Winsconsin Genetics Computer Group (UWGCG)) in the regions which are common to all three sequences (Figure 10). RT-PCR analysis of RNA isolated from leaves, roots, flowers and siliques showed that DZ2AT3 was specifically expressed in siliques. Southern hybridisation analysis showed that the DZ2AT3, DZ2 and DZ2B probes each identify a single identical band in A.thaliana. This indicates that A.thaliana contains one DZ2 gene in contrast to B. napus which contains two.

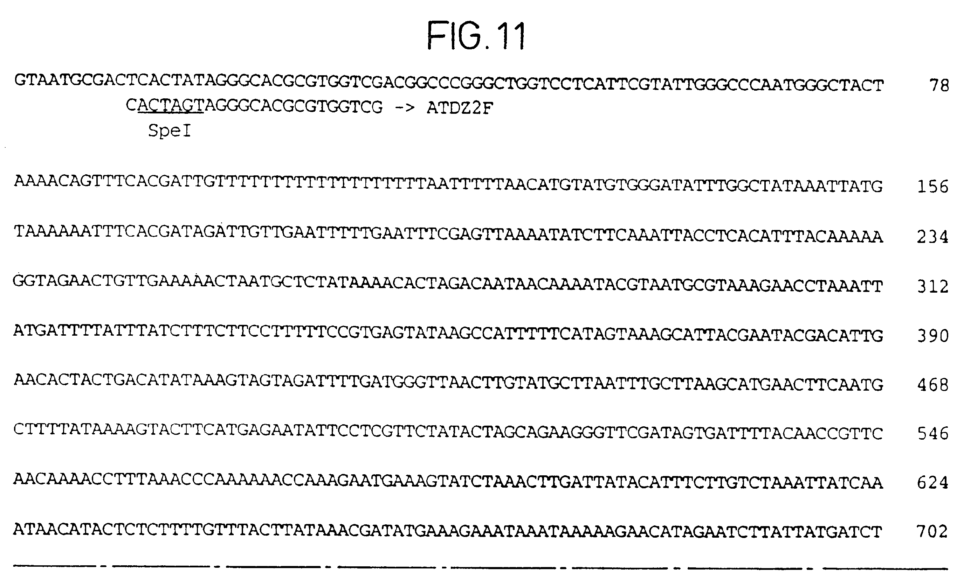

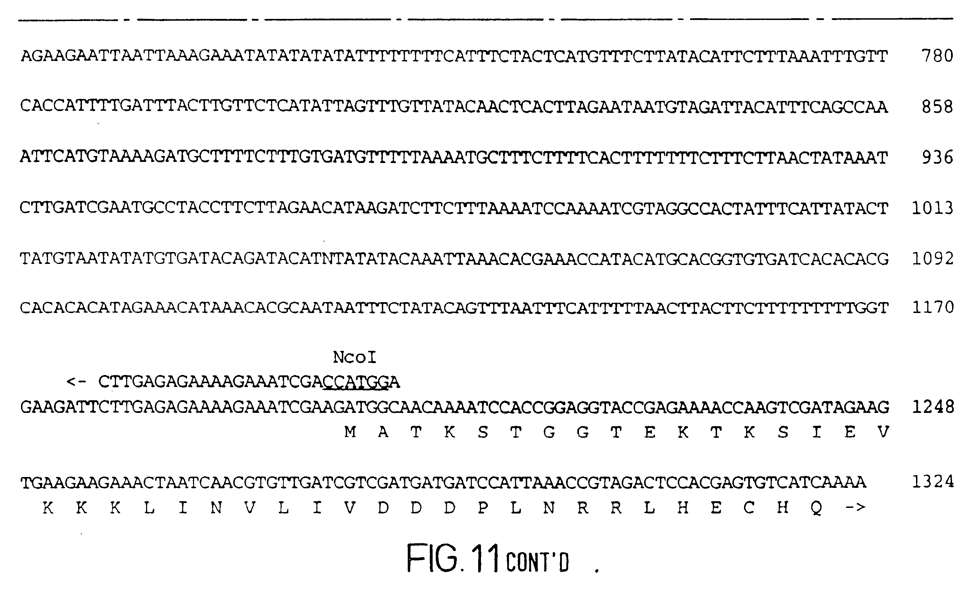

The Genome walker kit (Clonetech) was used to isolate the DZ2AT3 promoter from A.thaliana genomic DNA. Nested PCR was performed using primer GW1 first, then AT3GW2 each in conjunction with the Genome Walker kit primer (Figure 9). Figure 11 shows the sequence of the promoter region of DZ2AT3 thus obtained. The primers ATDZ2F and ATDZ2R were used to PCR a 1195bp promoter fragment from the DZ2AT3 genomic sequence (Figure 11).

| | |
|---|---|
| 5' CACTAGTAGGGCACGCGTGGTCG 3' | ATDZ2F |
| 5' TCCATGGTCGATTTCTTTTCTCTCAAG 3' | ATDZ2R |

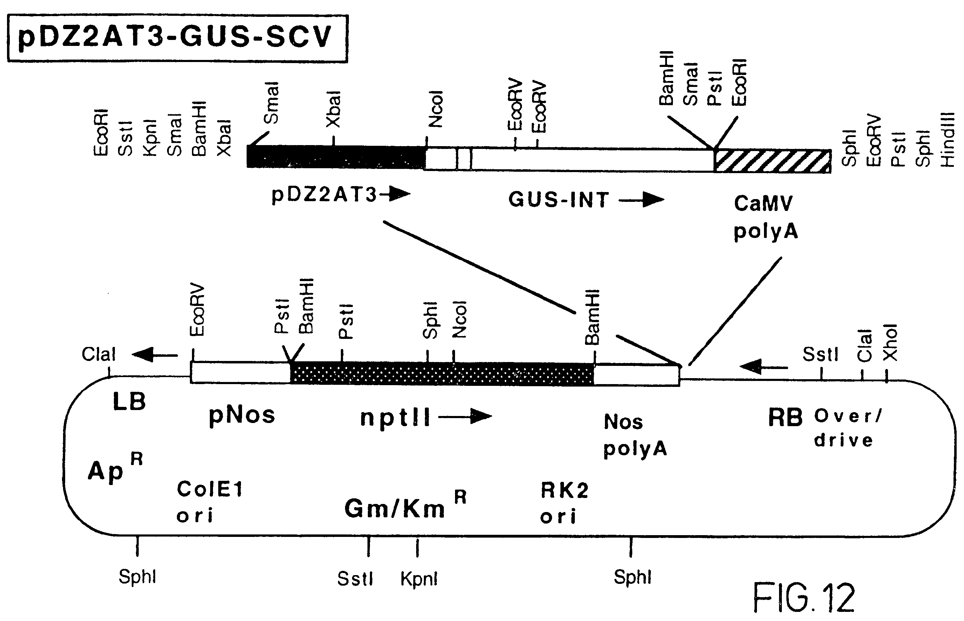

These primers introduced an SpeI site at the 5' end of the PCR fragment and an NcoI site around the initiating Met of DZ2AT3. Thus the PCR fragment was cloned as an SpeI, NcoI fragment between the XbaI and NcoI sites of pWP272 (WO 99/13089) forming pDZ2AT3-GUS. The chimeric pDZ2AT3-GUS-CaMV polyA gene was then transferred as a SaII, XhoI fragment into the SaII site of pSCV nos-nptII (WO 95/20668) forming pDZ2AT3-GUS-SCV (Figure 12). The pDZ2AT3-GUS-SCV binary vector was transferred to the agrobacterial strain pGV2260 and transformed B.napus plants produced by agrobacterial transformation essentially as described in Moloney M et al., (1989). Gus expression is observed in the pod DZ.

### Example 4 - Production of shatter-resistant B.napus plants by antisense downregulation of DZ2

Downregulation of the DZ2 gene or reduction in DZ2 protein levels in the pod DZ will result in plants that are resistant (or more resistant than without this modification) to pod shatter. Standard techniques, commonplace in the art, such as the expression of antisense DZ2 mRNA, full sense mRNA, partial sense mRNA or a ribozyme directed against DZ2 mRNA are effective. Expression of these RNAs requires a promoter that is active in the pod DZ at the time at which DZ2 is expressed. Ideally the promoter will be pod DZ-specific, however a useful promoter may be pod-specific or even constitutively active. A suitable promoter would be that of DZ2. Although DZ2 is expressed in the anther DZ. pod DZ and funiculus DZ, DZ2 promoter -GUS fusion studies show that in different transformants the relative level of expression in these three sites is variable but is stability hereditable. Thus some transformants are obtained in which expression is largely or exclusively confined to the pod DZ. This suggests that the pDZ2 promoter is comprised of distinct elements each specifying expression in a particular DZ. Alternatively the site of transgene integration may influence relative expression levels in the DZ tissues. The DZ2 promoter is therefore linked to the DZ2 cDNA such that the DZ2 is in the antisense orientation forming pDZ2-antiDZ2. This chimeric gene is transferred to the binary vector pNos-NptII-SCV (WO 96/30529). This binary vector is transferred to the agrobacterial strain pGV2260 and transformed *B.napus* plants produced by agrobacterial transformation essentially as described in Moloney M et al., (1989) Plant Cell Reports 8, 238-242. A proportion of transformed *B.napus* plants exhibit reduced levels of DZ2 message and are resistant to pod shatter.

### Example 5 - Production of shatter-resistant B.napus plants by antisense downregulation of DZ2

Downregulation of the DZ2 gene or reduction in DZ2 protein levels in the pod DZ will result in plants that are resistant to pod shatter. Techniques such as the expression of antisense DZ2 mRNA, full sense mRNA, partial sense mRNA or a ribozyme directed against DZ2 mRNA will be effective. Expression of these RNAs requires a promoter that is active in the pod DZ at the time at which DZ2 is expressed. Ideally the promoter will be pod DZ-specific, however a useful promoter may be pod-specific or even constitutively active. Although DZ2 / DZ2B is expressed in the anther DZ, pod DZ and funiculus DZ, DZ2B promoter -GUS fusion studies show that in different transformants the relative level of expression in these three sites is variable but is stably hereditable. Thus some transformants are obtained in which expression is largely or exclusively confined to the pod DZ. This suggests that the pDZ2 promoter is comprised of distinct elements each specifying expression in a particular DZ. Alternatively the site of transgene integration may influence relative expression levels in the DZ tissues. Thus a suitable DZ-specific promoter would be that of DZ2. DZ2B, DZ2AT3 or ESJ2A (WO 99/13089).

The primers DZ2FLA and DZ2RLA were used to PCR a 349bp fragment from the DZ2 cDNA:-

| | |
|---|---|
| 5'GGCGAATTCCGGTGAGGAGGCAGTAATC 3' | DZ2FLA |
| 5'GGCCCATGGCATACATACACACTTAGAC 3' | DZ2RLA |

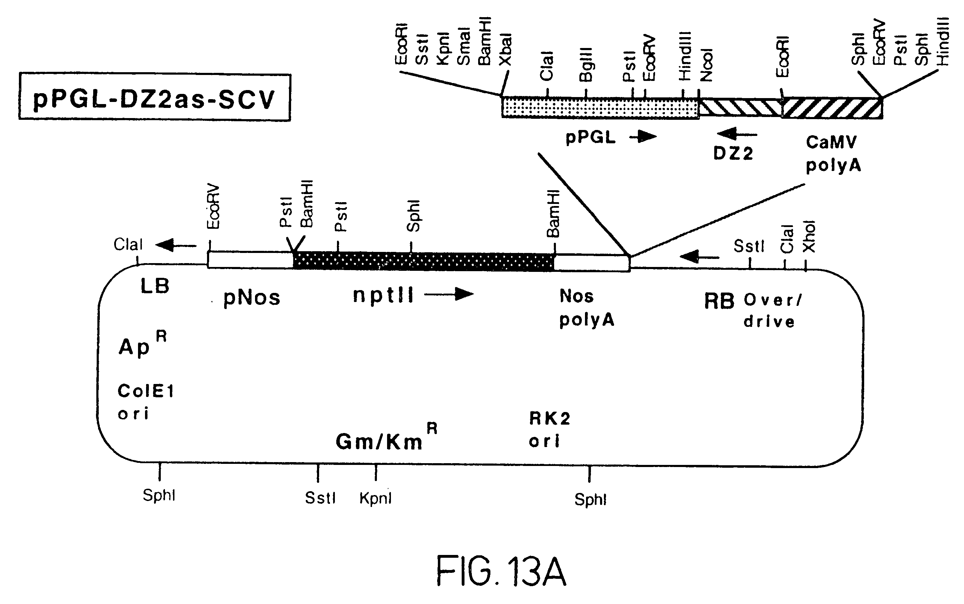

The primers introduce an EcoRI and NcoI site at the ends of the DZ2 PCR fragment. To link the DZ2 PCR fragment in an antisense orientation to the promoter of ESJ2A (pPGL) the DZ2 PCR fragment was cloned as a NcoI, EcoRI fragment between the NcoI and EcoRI sites of pWP272 (WO 99/13089) forming pPGL-DZ2as. The pPGL-antisense DZ2 chimeric gene was transferred as a XbaI, XhoI fragment from pDZ2as into the XbaI and SaII sites of the binary vector pSCV nos-nptII (WO 95/20668) forming pPGL-DZ2as-SCV (Figure 13a).

The pPGL-DZ2as-SCV binary vector was transferred to the agrobacterial strain pGV2260 and transformed B.napus plants produced by agrobacterial transformation essentially as described in Moloney M et al., (1989). A proportion of transformed B.napus plants exhibit reduced levels of DZ2 and DZ2B message and were resistant to pod shatter

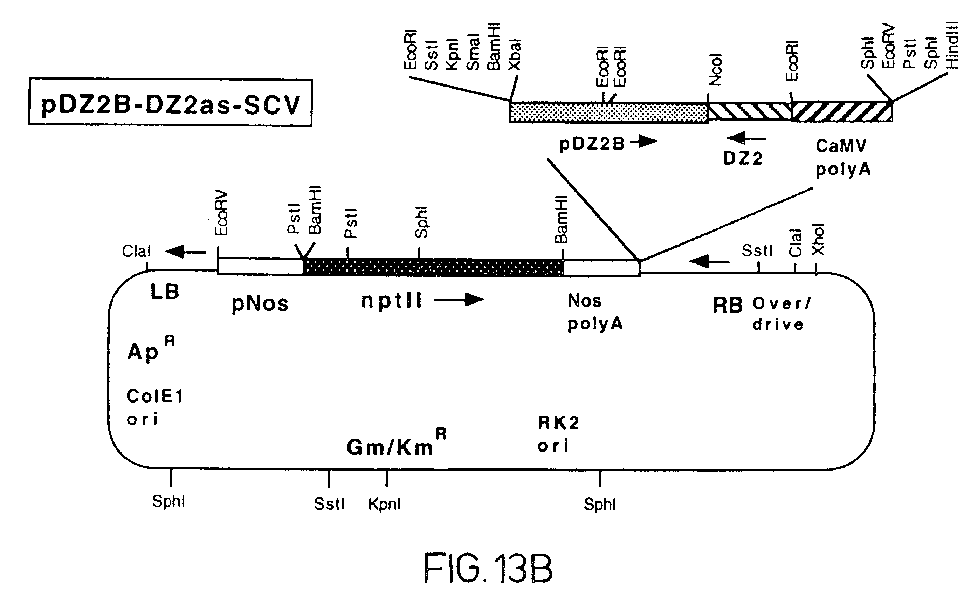

Similarly, to link the DZ2 PCR fragment in an antisense orientation to the promoter of DZ2B. the DZ2 PCR fragment is cloned as a NcoI. EcoRI fragment between the Ncol and EcoRI sites of pDZ2B-GUS forming pDZ2B-DZ2as. The pDZ2B-DZ2as chimeric gene is transferred as a XbaI. XhoI fragment from pDZ2B-DZ2as into the XbaI and SaII sites of the binary vector pSCV nos-nptII (WO 95/20668) forming pDZ2B-DZ2as-SCV (Figure 13b).

The pDZ2B-DZ2as-SCV binary vector is transferred to the agrobacterial strain pGV2260 and transformed B.napus plants. Again a proportion of transformed B.napus plants exhibit reduced levels of DZ2 and DZ2B message and are resistant to pod shatter.

Similarly a proportion of B.napus plants transformed with a pDZ23A-DZ2as-SCV construct exhibit reduced levels of DZ2 and DZ2B message and are resistant to pod shatter.

### Example 6 - Production of shatter-resistant B.napus plants by antisense downregulation of multiple DZ-expressed genes

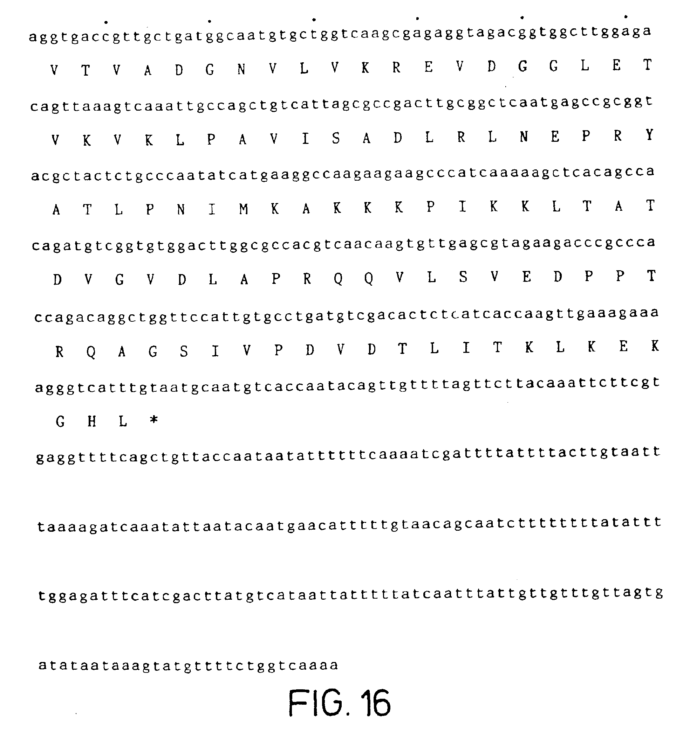

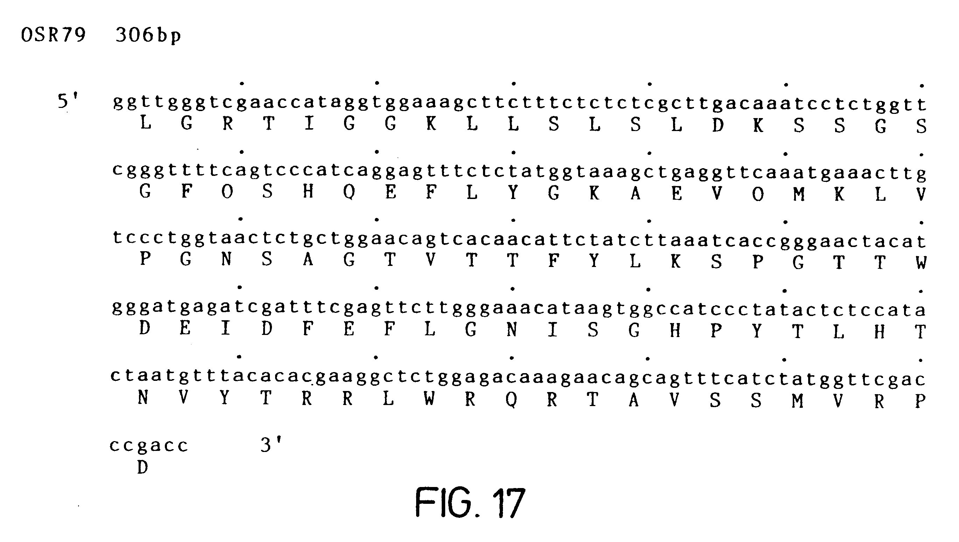

In addition to DZ2 several other DZ-expressed genes have been previously isolated and individually downregulated to result in B.napus plants that have increased resistance to pod shatter; namely Sac66 (WO 96/30529 Figure 15), DZ 15 (Figure 16) and OSR 7(9) (Figure 17). It is anticipated that downregulation of more than one gene involved in pod shatter will further increase resistance to pod shatter. This could be achieved by combining different transgenes by transformation with several transgenes each designed to downregulate a different DZ-expressed gene or by crossing together B.napus lines that individually are transformed with such transgenes. Such methods are complex either involving transformation with a construct containing multiple chimeric genes or require the maintenance of several transgenic loci in the breeding program. A preferred method is to transform with a chimeric gene consisting of a single promoter driving expression of an antisense or partial sense transcript which is comprised of elements of all the DZ-expressed genes to be downregulated. Similarly a single promoter could be used to drive the expression of multiple ribozymes each targeted against a different DZ-expressed gene. The use of a single promoter to drive expression of a combination of antisense, partial sense and ribozymes is also possible. Ideally the promoter will le pod DZ-specific, however a useful promoter may be pod-specific or even constitutively active. A suitable DZ-specific promoter would be that of DZ2, DZ2B, DZ2AT3 or ESJ2A.

Consequently the ESJ2A promoter was linked to a multiple antisense gene consisting of elements of Sac66, DZ2. DZ15 and OSR 7(9) in the following manner:- The original DZ 15 PCR product in pCRII (Invitrogen) (see Figure 16) was cloned as an EcoRI fragment into pBluescript SK (Stratagene) forming pDZ15-BS. such that the DZ15 3' end is nearest the SstI site of the vector. T7 and DZ 15RL primers were used to PCR a 456bp DZ15 fragment from pDZ15-BS which was cloned into the EcoRV site of pGEM5zf (Promega) forming pWP351, such that the DZ15 3' end is nearest the SphI site of the vector.

| | |
|---|---|
| 5' AATACGACTCACTATAGG 3' | T7 |
| 5' AACAGCTGAAAACCTCACGAAG 3' | DZ 15RL |

The EcoRI, NcoI fragment of pWP351 cloned between the EcoRI and NcoI sites of pDZ2-BS forming pWP356. pDZ2-BS consists of the DZ2 cDNA cloned as an EcoRI, XhoI fragment into pBluescript SK such that the 3' end is nearest the KpnI site of the vector. A 361bp Sac66 fragment was PCRed from the Sac66 cDNA (WO 96/30529) using the primers F1 and RI which introduce NcoI and PstI sites into the ends of the PCR product.

| | |
|---|---|
| 5'GGCCCATGGCTGCCAAGCTTTGAGTAGC 3' | F1 |
| 5'GGCCTGCAGTGCCTAGGATCTGGAAGC 3' | RI |

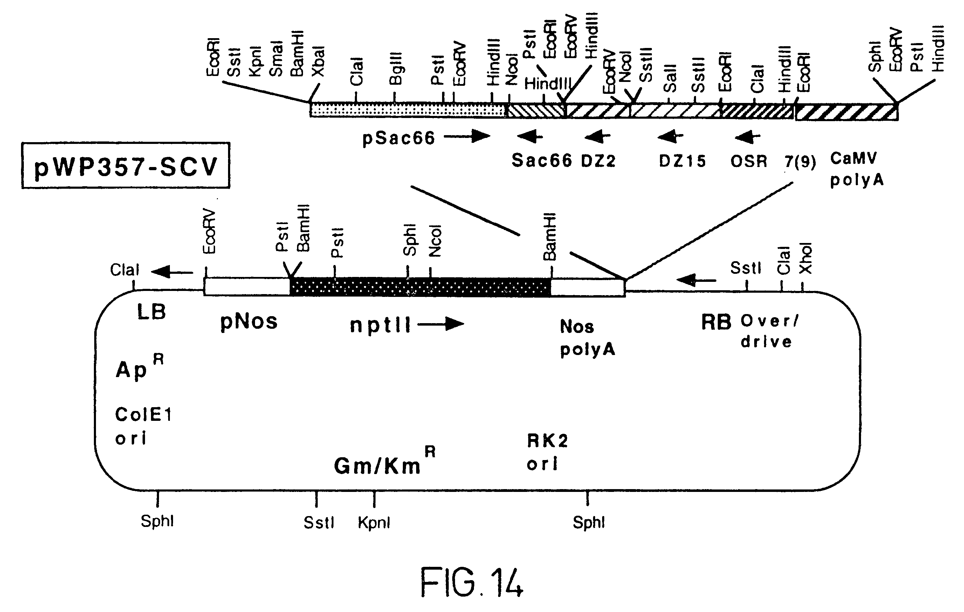

The Sac66 PCR product was cloned as an NcoI. EcoPI fragment between the NcoI and EcoRI sites of pWP272 (WO 99/13089) forming pWP288A. EcoRI DZ15+DZ2 and OSR 7(9) fragments from pWP356 and pOSR 7(9)-CRII were cloned into the EcoRI site of pWP288A such that DZ15 +DZ2 and OSR 7(9) are in an antisense orientation with respect to PGL promoter. (pOSR 7(9)-CRII consists of the 306bp OSR 7(9) PCR fragment (see Figure 17) cloned into pCRII (Invitrogen)). The chimeric pPGL-antisense Sac66+DZ2+Dz15+OSR 7(9) gene was transferred as a XbaI. XhoI fragment into the Xbal and SalI sites of the binary vector pSCV nos-nptII (WO 95/20668) forming pWP357-SCV (Figure 14). The pWP357-SCV binary vector was transferred to the agrobacterial strain pGV2260 and transformed B.napus plants produced by agrobacterial transformation essentially as described in Moloney M et al., (1989) Plant Cell Reports 8, 238-242.

Resistance to podshatter was measured using an impact pendulum device (Liu X-Y. Macmillan RH and Burrow RP 1994 Journal of Texture Studies 25 p179-189) (Table1). The mean energy values shown in Table 1 represent the energy required to rupture the pod on impact with the pendulum. These values are an average from measurements of 20 mature pods. The letters A to L indicate grouping of transformants with significantly different podshatter resistance (ie Group A is significantly different from B when analysed by ANOVA using a Fisher PLSD analysis with a significance level of 95 % (Statview 512+). Lines with a number of letters are not significantly different from other lines sharing the same letter. The results shown in Table 1 indicate that 24 lines exhibited significantly higher resistance to podshatter than non-transformed controls whilst 17 lines were not significantly different from the control. The degree and frequency of pod shatter resistance achieved with pWP357-SCV was greater than that obtained by transformation with constructs that downregulate a single DZ-expressed gene.

**Table 1**

| PLANTID MEAN ENERGY | | | | | | | | | | | | | |
|---|---|---|---|---|---|---|---|---|---|---|---|---|---|
| A213-24 | 6.752 | A | | | | | | | | | | | |
| A213-53 | 5.203 | | B | | | | | | | | | | |
| A213-34 | 3.864 | | | C | | | | | | | | | |
| A213-21 | 3.673 | | | C | | | | | | | | | |
| A213-4 | 3.54 | | | C | | | | | | | | | |
| A213-61 | 3.516 | | | C | | | | | | | | | |
| A214-30 | 3.46 | | | C | | | | | | | | | |
| A214-27 | 3.397 | | | C | | | | | | | | | |
| A213-9 | 3.277 | | | C | | | | | | | | | |
| A213-70 | 3.271 | | | C | D | | | | | | | | |
| A214-13 | 3.182 | | | C | D | E | | | | | | | |
| A213-11 | 3.182 | | | C | D | E | F | | | | | | |
| A213-69 | 3.005 | | | | D | E | F | G | | | | | |
| A213-60 | 2.945 | | | | D | E | F | G | H | | | | |
| A214-7 | 2.643 | | | | D | E | F | G | H | I | | | |
| A213-64 | 2.687 | | | | D | E | F | G | H | I | | | |
| A214-14 | 2.613 | | | | D | E | F | G | H | I | J | | |
| A213-25 | 2.581 | | | | D | E | F | G | H | I | J | | |
| A213-9 | 2.547 | | | | | E | F | G | H | I | J | | |
| A213-42 | 2.442 | | | | | | F | G | H | I | J | | |
| A213-31 | 2.431 | | | | | | | G | H | I | J | | |
| A214-10 | 2.42 | | | | | | | | H | I | J | | |
| A213-38 | 2.295 | | | | | | | | H | I | J | | |
| A214-8 | 2.26 | | | | | | | | H | I | J | | |
| A213-19 | 2.213 | | | | | | | | H | I | J | | |
| A214-25 | 2.128 | | | | | | | | | I | J | K | |
| A213-38 | 2.059 | | | | | | | | | I | J | K | |
| A213-16 | 2.005 | | | | | | | | | I | J | K | |
| A213-63 | 1.923 | | | | | | | | | I | J | K | |
| A213-33 | 1.91 | | | | | | | | | | J | K | L |
| A213-32 | 1.901 | | | | | | | | | | J | K | L |
| A213-37 | 1.791 | | | | | | | | | | J | K | L |
| RV27CONT | 1.787 | | | | | | | | | | | K | L |
| A213-10 | 1.623 | | | | | | | | | | | K | L |
| A213-58 | 1.595 | | | | | | | | | | | | L |
| A214-24 | 1.55 | | | | | | | | | | | | L |
| A213-3 | 1.518 | | | | | | | | | | | | L |
| A213-27 | 1.495 | | | | | | | | | | | | L |
| A213-40 | 1.395 | | | | | | | | | | | | L |
| A213-47 | 1.315 | | | | | | | | | | | | L |
| A213-43 | 1.286 | | | | | | | | | | | | L |
| A213-30 | 1.241 | | | | | | | | | | | | L |
| | | | | | | | | | | | | | L |

### REFERENCES

1. Bleasby A J and Attwood T K, OWL - A Non-redundant Composite Protein Sequence Database. Nucleic Acid Research 22:3574-3577 (1994)
2. Christoffersen and Laties, Proc. Natl. Acad. Sci. 79, 4060-4063 (1982)
3. Dean et al., EMBO J. 4: 3055-3061 (1985)
4. Deveraux et al., Nucl. Acids Res. 12:387 (1984)
5. Hocking P J and Mason L: Accumulation, distribution and redistribution of dry matter and mineral nutrients in fruits of canola (oilseed rape) and the effect of nitrogen fertiliser and windrowing. Australian Journal or Agriculture Research 44: 1377-1388 (1993)
6. Hakwa et al., Science 198:105-63 (1977)
7. Jenkins et al: 5th International Conference of Plant Molecular Biology, Abstract 310 (1997)
8. Johnson-Flanagan AM and Spencer MS: Ethylene production during development of mustard (Brassica juncea) and canola (Brassica napus) seed. Plant Physiol 106: 601-606 (1994)
9. Liang P and Pardee A: Differential display of eukaryotic messenger RNA by means of the polymerase chain reaction. Science 257: 967-971 (1992)
10. Laing P, Bauer D, Averboukh L, Warthoe P, Rohrwild M, Muller H, Strauss M, Pardee A B: Analysis of altered gene expression by differential display. Methods in Enzymol 254: 304-321 (1995)
11. Meakin P J and Roberts J A: Dehiscence of fruit in oilseed rape (Brassica napus L.): anatomy of pod dehiscence. J Expt. Bot 41: 995-1002 (1990a)
12. Meakin P J and Roberts J A: Dehiscence of fruit in oilseed rape (Brassica napus L.): the role of cell wall degrading enzymes and ethylene. J Expt. Bot 41: 1003-1011 (1990b)
13. Moloney M et al; Plant Cell Reports, 8, 238-242 (1989)
14. Needleman and Wunsch J. Mol. Biol. 48:443 (1970)
15. Smith and Waterman Adv. Appl. Math 2: 482 (1981)

## Claims

1. Isolated nucleic acid encoding a protein wherein the protein:
a) comprises the amino acid sequence shown in Figure 1 or;
b) has one or more amino acid deletions, insertions or substitutions relative to a protein as defined in a) above and has at least 95% amino acid sequence identity therewith, wherein said protein is involved in dehiscence.

2. Nucleic acid as claimed in claim 1 which comprises the sequence set out in Figure 1.

3. Nucleic acid which is antisense to nucleic acid as claimed in any one of claims 1 to 2.

4. Nucleic acid as claimed in any one of claims 1 to 3 including a promoter or other regulatory sequence which controls expression of said nucleic acid.

5. Nucleic acid as claimed in any one of claims 1 to 4 which is in the form of a vector.

6. A cell transformed with a nucleic acid as claimed in any one of claims 1 to 5.

7. A plant cell as claimed in claim 6.

8. A process for obtaining a cell as claimed in claim 6 or 7 comprising introducing nucleic acid as claimed in any one of claims 1 to 5 into said cell.

9. A plant or a part thereof comprising a cell as claimed in claim 6 or claim 7.

10. Propagating material or a seed comprising a cell as claimed in claim 6 or claim 7.

11. A process for obtaining a plant or plant part as claimed in claim 9 or claim 10 comprising obtaining a cell as claimed in claim 6 and growth thereof or obtaining a plant, plant part, or propagating material as claimed in claim 9 or claim 10 and growth thereof.

12. An isolated or recombinant protein which:
a) comprises the amino acid sequence shown in Figure 1 or;
b) has one or more amino acid deletions, insertions or substitutions relative to a protein as defined in a) above, and has at least 95% amino acid sequence identity therewith, wherein said protein is involved in dehiscence.

13. A process for producing a shatter-resistant *Brassica napus* plant or a part thereof, the process comprising transforming a *Brassica napus* plant or part thereof, with a nucleic acid which is antisense to nucleic acid as claimed in any one of claims 1 to 2.

14. A process as claimed in claim 13 which comprises obtaining a plant cell as claimed in claim 7 or part of a plant as claimed in claim 9 and deriving a plant therefrom.

15. A process as claimed in claim 13 which comprises obtaining propagating material or a seed as claimed in claim 10 and deriving a plant therefrom.

16. A process as claimed in claim 13 wherein the dehiscence is of a pod or of an anther.

17. Use of nucleic acid as claimed in claim 3 in the regulation/control of plant dehiscence.

18. Use of nucleic acid as claimed in any one of claims 1 to 5 as a probe.

## Patentansprüche

1. Isolierte Nukleinsäure, die ein Protein codiert, wobei das Protein:
a) die in Figur 1 gezeigte Aminosäuresequenz umfasst, oder
b) eine oder mehrere Aminosäure-Deletionen, -Insertionen oder -Substitutionen im Vergleich zu einem Protein aufweist, wie es in a) oben definiert ist, und mindestens 95% Aminosäuresequenzidentität damit hat, wobei das Protein an der Dehiszenz beteiligt ist.

2. Nukleinsäure nach Anspruch 1, umfassend die in Figur 1 aufgeführte Sequenz.

3. Nukleinsäure, die zur Nukleinsäure nach einem der Ansprüche 1 bis 2 antisense ist.

4. Nukleinsäure nach einem der Ansprüche 1 bis 3, einschließlich eines Promotors oder einer anderen regulatorischen Sequenz, die die Expression der Nukleinsäure steuert.

5. Nukleinsäure nach einem der Ansprüche 1 bis 4, die in der Form eines Vektors ist.

6. Zelle, die mit einer Nukleinsäure nach einem der Ansprüche 1 bis 5 transformiert ist.

7. Pflanzenzelle nach Anspruch 6.

8. Verfahren zum Gewinnen einer Zelle nach Anspruch 6 oder 7, umfassend das Einbringen einer Nukleinsäure nach einem der Ansprüche 1 bis 5 in die Zelle.

9. Pflanze oder Teil davon, umfassend eine Zelle nach Anspruch 6 oder Anspruch 7.

10. Fortpflanzungsmaterial oder ein Samen, umfassend eine Zelle nach Anspruch 6 oder Anspruch 7.

11. Verfahren zum Gewinnen einer Pflanze oder eines Pflanzenteils nach Anspruch 9 oder Anspruch 10, umfassend das Gewinnen einer Zelle nach Anspruch 6 und ihre Anzucht, oder Gewinnen einer Pflanze, eines Pflanzenteils oder eines Fortpflanzungsmaterials nach Anspruch 9 oder Anspruch 10 und deren Anzucht.

12. Isoliertes oder rekombinantes Protein, das:
a) die in Figur 1 gezeigte Aminosäuresequenz umfasst oder
b) eine oder mehrere Aminosäure-Deletionen, -Insertionen oder -Substitutionen im Vergleich zu einem Protein aufweist, wie es in a) oben definiert ist, und mindestens 95% Aminosäuresequenzidentität damit hat, wobei das Protein an der Dehiszenz beteiligt ist.

13. Verfahren zum Herstellen einer erschütterungsresistenten *Brassica napus*-Pflanze oder eines Teils davon, wobei das Verfahren das Transformieren einer *Brassica napus*-Pflanze oder eines Teils davon, mit einer Nukleinsäure, die antisense zur Nukleinsäure nach einem der Ansprüche 1 bis 2 ist, umfasst.

14. Verfahren nach Anspruch 13, umfassend das Gewinnen einer Pflanzenzelle nach Anspruch 7 oder eines Teils einer Pflanze nach Anspruch 9, und Herleiten einer Pflanze daraus.

15. Verfahren nach Anspruch 13, umfassend das Gewinnen von Fortpflanzungsmaterial oder eines Samens nach Anspruch 10 und Herleiten einer Pflanze daraus.

16. Verfahren nach Anspruch 13, wobei die Dehiszenz aus einer Schote oder einer Anthere stammt.

17. Verwendung einer Nukleinsäure nach Anspruch 3 bei der Regulation/Steuerung der Pflanzen-Dehiszenz.

18. Verwendung einer Nukleinsäure nach einem der Ansprüche 1 bis 5 als Sonde.

## Revendications

1. Acide nucléique isolé codant pour une protéine, ladite protéine :
a) comprenant la séquence d'acides aminés représentée dans la figure 1 ; ou
b) ayant une ou plusieurs délétions, insertions ou substitutions d'acides aminés par rapport à une protéine telle que définie en a) ci-dessus et ayant une identité de séquence d'acides aminés d'au moins 95 % avec celle-ci,
ladite protéine étant mise en jeu dans la déhiscence.

2. Acide nucléique selon la revendication 1 qui comprend la séquence présentée dans la figure 1.

3. Acide nucléique qui est antisens par rapport à l'acide nucléique selon l'une quelconque des revendications 1 à 2.

4. Acide nucléique selon l'une quelconque des revendications 1 à 3 comprenant un promoteur ou une autre séquence régulatrice qui contrôle l'expression dudit acide nucléique.

5. Acide nucléique selon l'une quelconque des revendications 1 à 4 qui est sous la forme d'un vecteur.

6. Cellule transformée avec un acide nucléique selon l'une quelconque des revendications 1 à 5.

7. Cellule végétale selon la revendication 6.

8. Procédé pour l'obtention d'une cellule selon la revendication 6 ou 7 consistant à introduire un acide nucléique selon l'une quelconque des revendications 1 à 5 dans ladite cellule.

9. Plante ou partie de celle-ci comprenant une cellule selon la revendication 6 ou la revendication 7.

10. Matériel de propagation ou graine comprenant une cellule selon la revendication 6 ou la revendication 7.

11. Procédé pour l'obtention d'une plante ou d'une partie de plante selon la revendication 9 ou la revendication 10 consistant à obtenir une cellule selon la revendication 6 et à faire croître celle-ci ou à obtenir une plante, une partie de plante ou un matériel de propagation selon la revendication 9 ou la revendication 10 et à faire croître celui-ci.

12. Protéine isolée ou recombinée qui :
a) comprend la séquence d'acides aminés représentée dans la figure 1 ; ou
b) a une ou plusieurs délétions, insertions ou substitutions d'acides aminés par rapport à une protéine telle que définie en a) ci-dessus et a une identité de séquence d'acides aminés d'au moins 95 % avec celle-ci,
ladite protéine étant mise en jeu dans la déhiscence.

13. Procédé pour la production d'une plante de type *Brassica napus* résistante à l'égrenage ou d'une partie de celle-ci, le procédé consistant à transformer une plante de type *Brassica napus* ou une partie de celle-ci, avec un acide nucléique qui est antisens par rapport à un acide nucléique selon l'une quelconque des revendications 1 à 2.

14. Procédé selon la revendication 13 qui consiste à obtenir une cellule végétale selon la revendication 7 ou une partie d'une plante selon la revendication 9 et à dériver une plante à partir de celle-ci.

15. Procédé selon la revendication 13 qui consiste à obtenir un matériel de propagation ou une graine selon la revendication 10 et à dériver une plante à partir de celui-ci.

16. Procédé selon la revendication 13 dans lequel la déhiscence est celle d'une cosse ou d'une anthère.

17. Utilisation d'un acide nucléique selon la revendication 3 dans la régulation de la déhiscence d'une plante/la lutte contre celle-ci.

18. Utilisation d'un acide nucléique selon l'une quelconque des revendications 1 à 5 comme sonde.
